(19) Europäisches Patentamt
European Patent Office
Office européen des brevets

(11) EP 0 667 345 B1

(12) EUROPEAN PATENT SPECIFICATION

(45) Date of publication and mention of the grant of the patent: **29.09.1999 Bulletin 1999/39**

(21) Application number: **95300883.6**

(22) Date of filing: **13.02.1995**

(51) Int Cl.[6]: **C07D 405/04**, A61K 31/415, A61K 31/34, A61K 31/38, C07D 491/04, C07D 495/04, C07D 231/56 // (C07D405/04, 307:00, 231:00), (C07D491/04, 307:00, 231:00), (C07D495/04, 333:00, 231:00)

# (54) 1-Benzyl-3-(substituted aryl)-condensed pyrazole derivatives as inhibitors of platelet aggregation

1-Benzyl-3(substituiertes Aryl)-kondensierte Pyrazolderivate als Inhibitoren der Blutplättchenaggregation

Dérivés 1-benzyle-3-(aryle substitué) du pyrazole condensée comme agents inhibiteurs de l'aggrégation plaquettaire

(84) Designated Contracting States:
**CH DE FR GB LI**

(30) Priority: **14.02.1994 JP 5094694**

(43) Date of publication of application:
**16.08.1995 Bulletin 1995/33**

(73) Proprietor: **Yung Shin Pharm. Ind. Co. Ltd.**
**Tachia, Taichung (TW)**

(72) Inventors:
- **Sheng-Chu, Kuo 5th Floor, Apt no 1**
  **Taichung (TW)**
- **Fang, Yu Lee**
  **Tachia Taichung (TW)**
- **Che-Ming, Teng**
  **Taipei (TW)**

(74) Representative: **Jones, Stephen Anthony et al**
**E. N. Lewis & Taylor**
**144 New Walk**
**Leicester LE1 7JA (GB)**

(56) References cited:
**EP-A- 0 254 241**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 667 345 B1

## Description

This invention relates to novel condensed pyrazole derivatives, in particular to 1-(benzyl)-3-(substituted aryl) condensed pyrazole derivatives.

Cardiovascular diseases, especially various forms of thrombosis, such as coronary, embolic, venous and traumatic thrombosis, account for a large number of death per year. In fact it is estimated by the American Heart Association that 54% of all deaths is the United States can be attributed to cardiovascular disease. It is therefore important for us to be familiar with physical, chemical and clinical aspects of drugs used to treat these form of thrombosis. Since it is believed that initiation of thrombus formation is dependent on platelet aggregation, inhibitors of platelet aggregation have potential as drugs that could more effectively combat thrombosis.

A number of inhibitors of platelet aggregation have been used clinically in the treatment and prevention of vascular thrombosis. These inhibitors may be divided in to several groups based on their mode of action, aspirin, dipyridamole, ticlopidine, and eicosa pentanoic acid (EPA) being used most frequently. However, the side effects of these inhibitors have prompted a search for novel compounds possessing more potent inhibiting activity on platelet aggregation.

EP-A-0254241 discloses certain 3-(3,5-di-tertiary butyl-4-hydroxyphenyl) -1H-pyrazolo [3,4-b] pyridine compounds. These compounds are said to exhibit inter alia inhibitory activity on platelet aggregation.

According to the present invention, there are provided l-benzyl-3-(substituted aryl) condensed pyrazoles of the general formula (A):

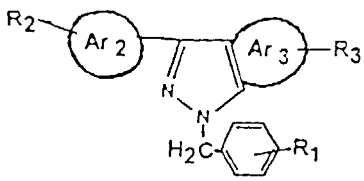

(A)

wherein

$R_1$ represents H, $C_{1-3}$alkyl, halogen, or -OR, in which R represents H or $C_{1-3}$ alkyl;

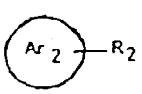 represents 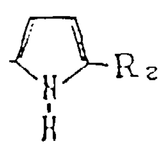 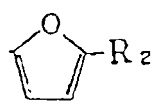 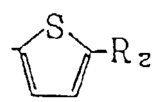 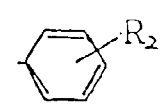

wherein

$R_2$ represents -COOR, $-CH_2OR$, H, $C_{1-3}$alkyl, or halogen, in which R is as defined above;

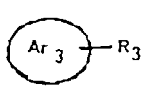 represents 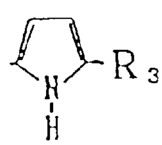 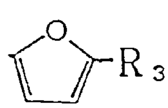 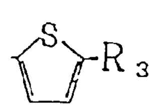 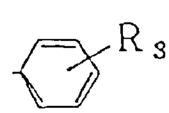

wherein

$R_3$ represents H, $C_{1-3}$alkyl, halogen, or -OR, in which R is as defined above;

and pharmaceutically acceptable salts thereof.

A preparation method for compounds (A) is shown in Fig.1.

Figure 1

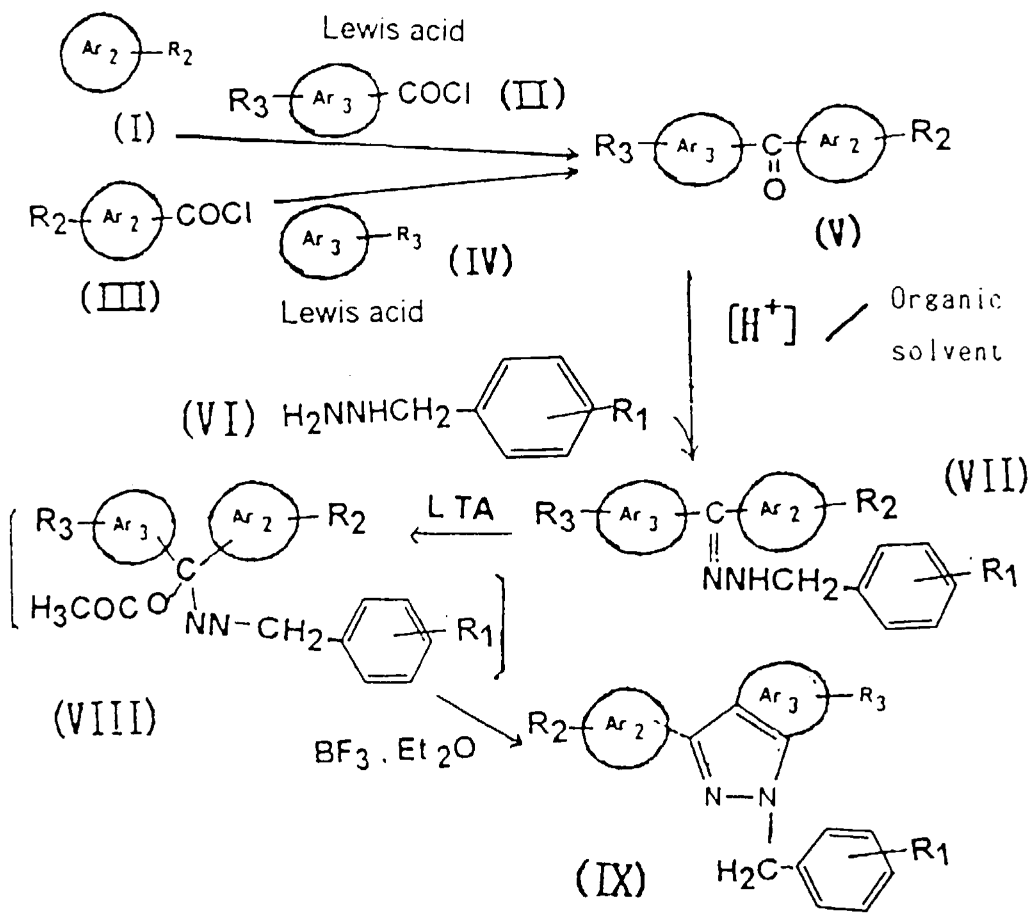


The method might be performed using substituted arylcarboxylic acid chlorides (II,III) as starting materials, which are treated with substituted aromatic compounds(I,IV) via a Friedel-Crafts reaction to form ketones (V), and then condensed with hydrazines (VI) to give the corresponding hydrazones(VII).

The hydrazones (VII) are further treated with lead tetraacetate and borontrifluoride-etherate according to Yushina, S. et al.(Yakugaku Zasshi Vol 97, 955 (1977), to give the 1-benzyl-3-(substituted aryl)-condensed pyrazoles (IX).

Hydrolysis of ester groups of compounds (IX) with acids or bases gives the corresponding carboxylic acid derivatives (X). Ester groups of compounds (IX) might be reduced with strong reducing agents, eg $LiAlH_4$ or $CaBH_4$ to hydroxymethyl groups to give corresponding alcohols (XI).

The structures of compounds of formula (A) described above were assigned on the basis of IR, NMR, MS, and elemental analytical data.

The pharmacological activity of these compounds was determined by turbidimetry according to Born, G.V.R. (J Physiol, Vol 168, 178, 1963). Based on the method samples were suspended in rabbit platelets which were washed with platelet-rich plasma. The aggregation was then counted by a Lumi-aggregometer (Model 1020, Paytoon, Canada). The results are shown in Table 1. Compounds of formula(A) at a concentration of 100 µg/ml were found to inhibit perfectly platelet aggregation induced by arachidonic acid(AA), collagen, ADP, and PAF.

The compounds of the invention can be converted into base salts by partial or complete neutralization with bases. Acid-addition salts can also be converted into the corresponding free compounds or inner salts thereof in an

analogous manner. Examples of such salts include inorganic and organic acid addition salts such as hydrochloride, hydrobromide, sulphate, phosphate, acetate, fumarate, tartrate, maleate, citrate, lactate, oxalate, or similar pharmaceutically acceptable inorganic and organic acid addition salts.

Pharmaceutical preparations according to the invention which contain compounds of formula (A) or pharmaceutically acceptable salts thereof may be administered enterally or parenterally and may contain the pharmaceutical active ingredient alone or together with one or more pharmaceutically acceptable adjuvants or carrier materials. Suitable carriers for oral dosage forms are, in particular, fillers, such as sugars, for example lactose, sucrose, mannitol, and binders, such as starch mucilage using, for example wheat, rice or potato starch, and/or, if desired, disintegrating agents or other adjuncts. Carriers for parenteral dosage forms are, in particular, aqueous solutions and lipophilic solvents or vehicles, such as fatty oils, and/or, if desired, viscosity-increasing substances, for example sodium carboxymethyl cellulose, and sorbitol. The preferred individual dosage is 50 to 300 mg for oral administration and 2 to 15mg for intravenous administration, which may typically be administered up to three times daily.

Particular preferred sub-groups of compounds of general formula (A) include those in which

a)

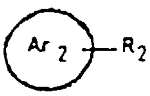 represents 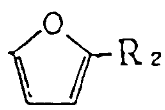 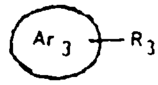 represents 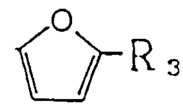

b)

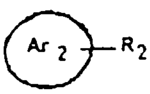 represents 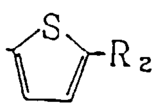 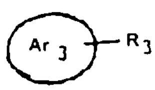 represents 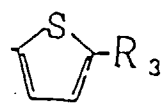

c)

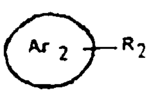 represents 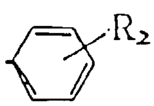 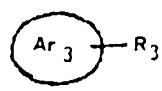 represents 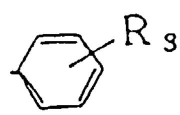

d)

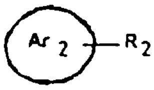 represents 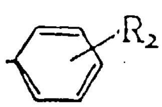 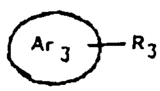 represents 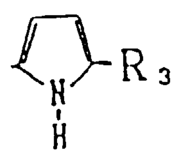

e)

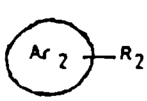 represents 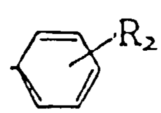 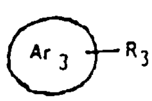 represents 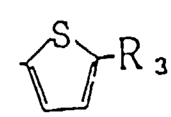

f)

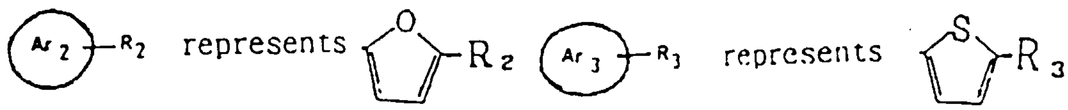
represents … represents

g)

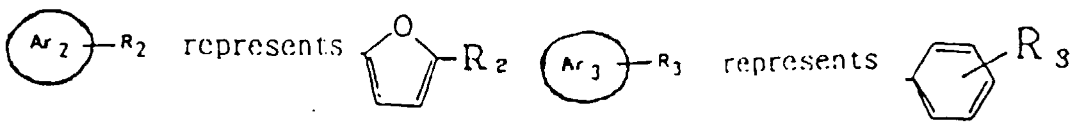
represents … represents

h)

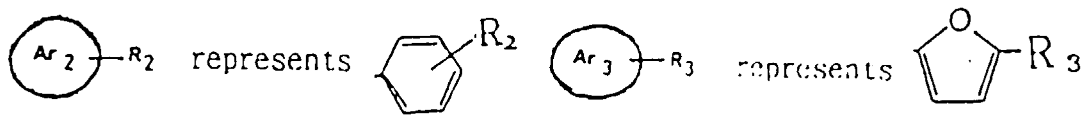
represents … represents

i)

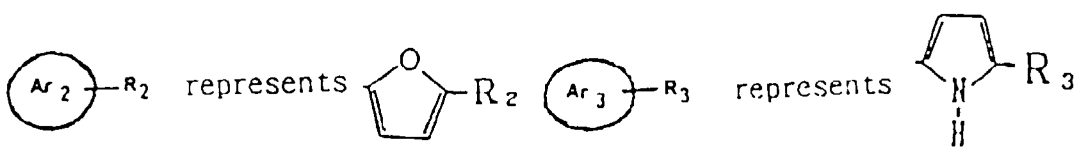
represents … represents

j)

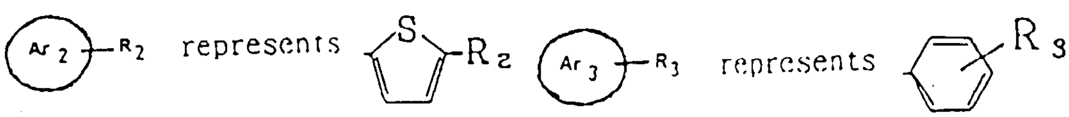
represents … represents

k)

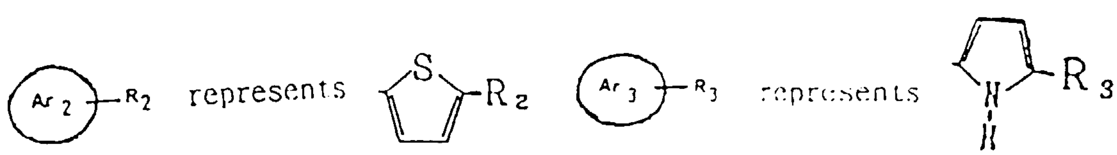
represents … represents

l)

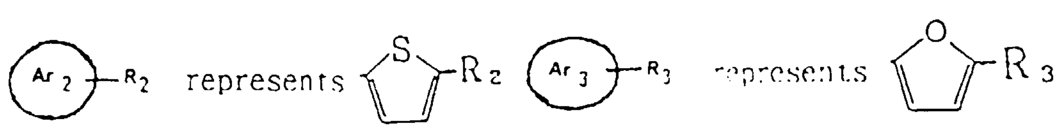
represents … represents

m)

$R_1$ represents $C_{1-3}$alkyl, halogen, or -OR;

$R_2$ represents -COOR, -$CH_2$OR, $C_{1-3}$alkyl, or halogen;
$R_3$ represents H, $C_{1-3}$alkyl, halogen, or -OR;

in which R represents $C_{1-3}$alkyl.

n)

$R_1$ represents $C_{1-3}$alkyl, or -OR;
$R_2$ represents -COOR, -$CH_2$OR, $C_{1-3}$alkyl, or halogen;
$R_3$ represents H, $C_{1-3}$alkyl, halogen, or -OR;

in which R represents $C_{1-3}$alkyl.

o)

$R_1$ represents H, halogen, or -OR;
$R_2$ represents -COOR, -$CH_2$OR, H, or halogen;
$R_3$ represents H, halogen, or -OR;

in which R represents $C_{1-3}$alkyl.

p)

$R_1$ represents H, or -OR;
$R_2$ represents -COOR, -$CH_2$OR, $C_{1-3}$alkyl, or halogen;
$R_3$ represents H, halogen, or -OR;

in which R represents $C_{1-3}$alkyl.

Preferred embodiments of the present invention will now be described, by way of illustration only, in the following synthetic examples.

Preparation of substituted aryl-substituted aryl ketones (V)

The preparation of intermediate ketones (V), in which $R_2$ represents -COOR (in which R represents $C_{1-3}$alkyl), H, or halogen, and $R_3$, $Ar_2$, $Ar_3$ are as defined in formula (A), is shown in Scheme 1.

Scheme 1

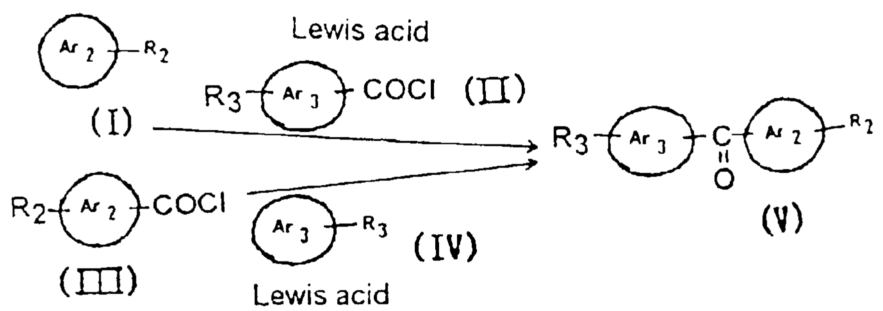


Substituted arylcarboxylic acid chlorides (II,III) are used as starting materials to acylate the substituted aromatic compounds (I,IV) in organic solvents in the presence of Lewis acid to form the corresponding ketones (V). The structures are determined using IR, NMR, MS, and elemental analytical data.

EXAMPLE I-1-1

5-Methoxycarbonyl-2-furyl phenyl ketone (V-1)

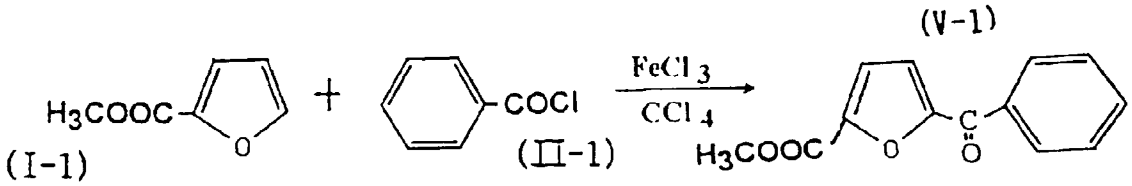


(I-1) (II-1) (V-1)

Compound V-1 was reported by Yushina,S. et al.(Yakugaku Zasshi Vol 97, 955, 1977). The synthesis is described as follows.

Anhydrous ferric chloride (0.42 g ,0.0026 mole) and benzoyl chloride (II-1) (29.65 g), were dissolved in $CCl_4$ (40 ml) and added dropwise with methyl furan-2-carboxylate(I-1) (24 g, 0.19 mole). The reaction mixture was then heated under refluxing for 16 hrs, and after cooling was added with water (120 ml). The mixture was extracted with $CCl_4$, then the $CCl_4$ layer was washed with water, 5% sodium bicarbonate solution, and then with water, till neutral, then dried over anhydrous magnesium sulfate and filtered. The solvent of the filtrate was evaporated under reduced pressure, the residue was recrystallized from isopropanol, and then from methanol to give compound V-1. Yield 18.75 g (42.9%).

mp: 70-73°C
MS(%), m/z: 230 (100) (M+).
IR(KBr) ν max : 1720, 1650 $cm^{-1}$ (C=O).
$^1$H-NMR ($CDCl_3$) δ :

3.86 (3H, s, $CH_3$),
7.26-7.32 (2H, m, $C_{3',5'}$-H),
7.40-7.65 (3H, m, $C_{3,4,4'}$-H),
8.05-8.10 (2H, m, $C_{2',6'}$-H).

| | | |
|---|---|---|
| Anal (for $C_{13}H_{10}O_4$) Calcd | C, 67.82; | H, 4.38. |
| Found | C, 67.60; | H, 4.21. |

EXAMPLE I-1-2

5-(Methoxycarbonyl)-2-furyl 4-methylphenyl ketone (V-2)

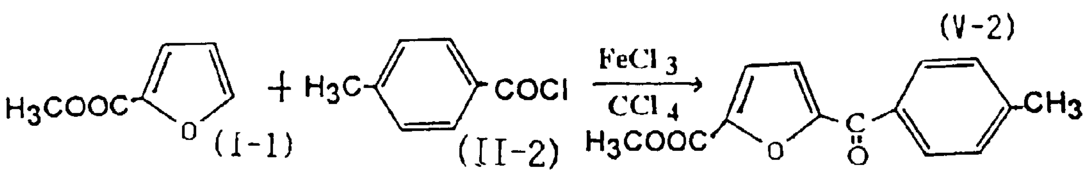


(I-1) (II-2) (V-2)

4-Methylbenzoyl chloride (II-2) (31 g, 0.20 mole) was used as the starting material, and treated according to the procedure described in example I-1-1 to give compound V-2. Yield, 17.0 g (36.7 %).

mp: 102-104°C.
MS(%),m/z: 244 (100) (M+).
IR(KBr) ν max : 1730, 1650 $cm^{-1}$(C=O).
$^1$H-NMR (CDCl3) δ:

2.45 (3H, S, $CH_3$), 3.95 (3H, S, $OCH_3$),
7.26-7.35 (4H, m, $C_{3,4,3',5'}$-H),
8.00 (2H, d, J=8.0 Hz, $C_{2',6'}$-H).

| | | |
|---|---|---|
| Anal (for $C_{14}H_{12}O_4$) Calcd | C, 64.61; | H, 4.95. |
| Found | C, 68.61; | H, 4.75. |

EXAMPLE I-1-3

5-Methoxycarbonyl-2-furyl 4'-methoxyphenyl ketone (V-3)

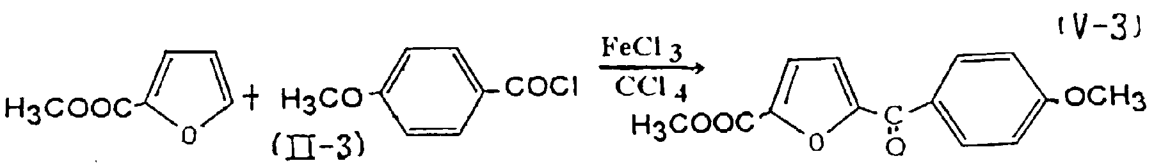


4-Methoxybenzoyl chloride (II-3) (35.7 g, 0.21 mole) was used as the starting material, and treated according to the procedure described in example I-1-1 to give compound V-3. Yield, 21.7 g (43.9%).

mp: 99-102°C.
MS(%),m/z: 260 (100) (M+).
IR(KBr) ν max : 1730, 1650 $cm^{-1}$(C=O).
$^1$H-NMR ($CDCl_3$)δ:

3.90(3H, S,-$OCH_3$), 3.96 (3H, S,-$COOCH_3$),
7.00 (2H, d, J=7.8 Hz, $C_{3',5'}$-H),
7.26-7.32 (2H, m, $C_{3,4}$-H),
8.15 (2H, d, J=7.8 Hz, $C_{2',6'}$-H).

| | | |
|---|---|---|
| Anal (for $C_{14}H_{12}O_5$) Calcd | C, 64.61; | H, 4.65. |
| found | C, 64.39; | H, 4.90. |

EXAMPLE I-1-4

4-Fluorphenyl5-methoxycarbonyl-2-furyl ketone(V-4)

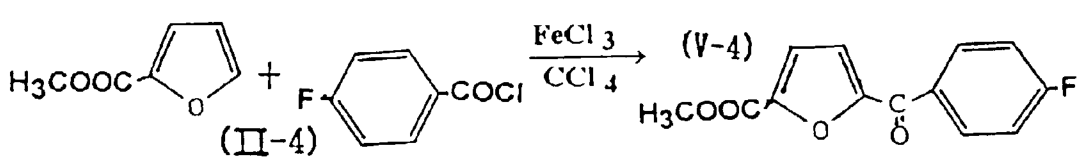

4-Fluorobenzoyl chloride (II-4) (33g, 0.21 mole) was used as the starting material, and treated according to the procedure described in example I-1-1 to give compound V-4. Yield, 27.4g (58.1%).

mp: 102-105°C.
MS(%),m/z: 236 (100) (M+).
IR(KBr)νmax : 1740, 1660 $cm^{-1}$(C=O).
$^1$H-NMR ($CDCl_3$)δ:

3.89 (3H, S, $CH_3$),
7.40-7.53 (4H, m, $C_{3,4,3',5'}$-H),
8.01-8.09 (2H, m, $C_{2',6'}$-H).

| | | |
|---|---|---|
| Anal (for $C_{13}H_9FO_4$: Calcd | C, 62.91; | H, 3.66. |
| Found | C, 61.20; | H, 3.90. |

EXAMPLE I-1-5

5-Methoxycarbonyl-2-furyl 2'-thienyl ketone(V-5)

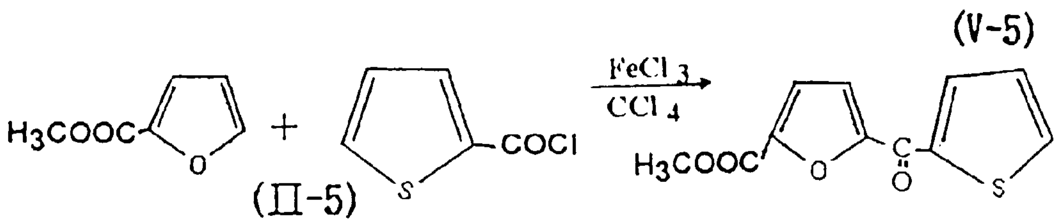


Thiophene-2-carboxylic acid chloride (II-5) (30.5g, 0.21mole) was used as the starting material, and treated according to the procedure described in example I-1-1 to give compound V-5. Yield, 28.7g (63.8%).

mp: 103-106°C.
MS(%),m/z: 236 (100) (M+).
IR(KBr)νmax : 1720, 1620 $cm^{-1}$(C=O).
$^1$H-NMR ($CDCl_3$)δ:

3.98 (3H, S, $CH_3$),
7.22-7.31 (2H, m, $C_{3,4}$-H),
7.41 (1H, d, J=3.5 Hz, $C_4$-H),
7.76 (1H, d, J=3.5 Hz, $C_3$-H),
8.36 (1H, d, J=4.5 Hz, $C_5$-H).

| | | |
|---|---|---|
| Anal (for $C_{11}H_8O_4S$) Calcd | C, 55.93; | H, 3.41. |
| Found | C, 55.71; | H, 3.23. |

EXAMPLE I-1-6

5-Methyl-2-furyl phenyl ketone(V-6)

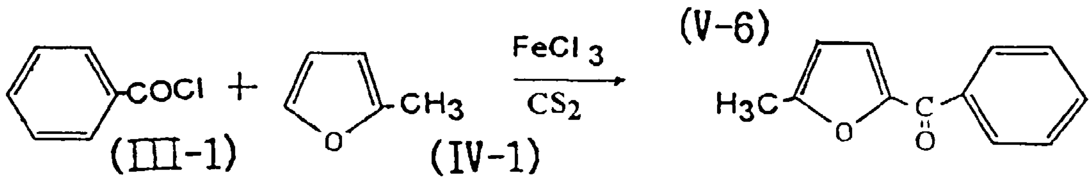


The compound V-6 was reported by S.Yushina et al (Yakugaku Zasshi Vol 97,955, 1977). The synthesis is described as follows.

Anhydrous ferric chloride (20g) and benzoyl chloride (III-1) (10.5g, 0.075 mole), were dissolved in carbon disulfide ($CS_2$) (100ml) and stirred at 20°C and added dropwise with a solution of 2-methylfuran (IV-1) (12.3g, 0.15 mole) in 100ml carbon disulfide. The reaction mixture under was heated refuxing. After cooling, the mixture was added slowly with 10% HCl (600ml) solution and then extracted with $CCl_4$. The $CCl_4$ layer was washed with water, 5% sodium bicarbonate solution, and then with water till neutral, then dried over anhydrous magnesium sulfate and filtered. The solvent of the filtrate was evaporated and the residue was purified by column chromatography (silica gel-benzene) to give compound V-6, as a colourless liquid. Yield, 7.0g (50%).

MS(%),m/z: 186 (100) (M+).
IR(KBr)νmax: 1700 $cm_{-1}$(C=O).
$^1$H-NMR ($CDCl_3$)δ:

2.37 (3H, S, $CH_3$),
6.13 (1H, d, J=3.5 Hz, $C_4$-H),
7.03 (1H, d, J=3.5 Hz, $C_3$-H),
7.20-7.90 (5H, m, phenyl-H).

EXAMPLE I-1-7

5-Methyl- 2-furyl p-methylphenyl ketone (V-7)

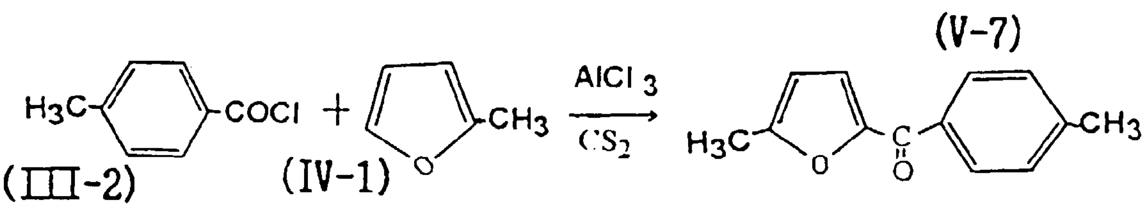


p-Methylbenzoyl chloride (III-2) (11.6g, 0.075 mole) was used as the starting material and treated according to the procedure described in example I-1-6 to give compound V-7, as a colourless liquid. Yield, 7.5g (50%).

MS(%),m/z: 200 (100) (M+).
IR(KBr)νmax: 1655 $cm^{-1}$(C=O).
$^1$H-NMR ($CDCl_3$)δ:

2.37 (3H, S, $C_5$-$CH_3$),

2.45 (3H, S, $C_4$-$CH_3$),
6.15 (1H, d, J=3.5 Hz, $C_4$-H),
7.02 (1H, d, J=3.5 Hz, $C_3$-H),
7.20 (2H, d, J=7.0 Hz, $C_{3',5'}$-H),
7.90 (2H, d, J=7.0 Hz, $C_{2',6'}$-H).

EXAMPLE I-1-8

p-Chlorophenyl 5-methyl-2-furyl ketone(V-8)

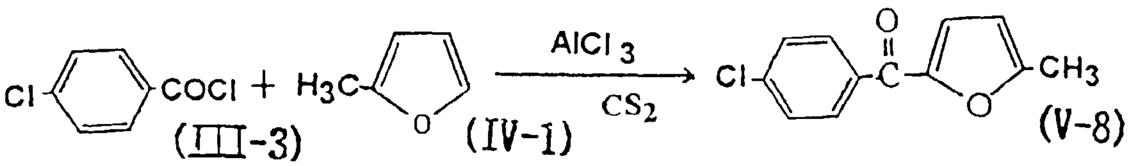


4-Chlorobenzoyl chloride (III-3) (13.1g, 0.075 mole) was used as the starting material and treated according to the procedure described in example I-1-6 to give compound V-8, as a colourless liquid. Yield, 10g (60%).

MS(%),m/z: 220 (100) (M+).
IR(KBr)νmax: 1680 $cm^{-1}$(C=O).
$^1$H-NMR ($CDCl_3$)δ:

2.40 (3H, S, $CH_3$),
6.18 (1H, d, J=3.5 Hz, $C_4$-H),
7.18 (1H, d, J=3.5 Hz, $C_3$-H),
7.35-7.85 (4H, m, phenyl-H).

EXAMPLE I-1-9

p-Chlorophenyl 5-methyl-2-thienyl ketone(V-9)

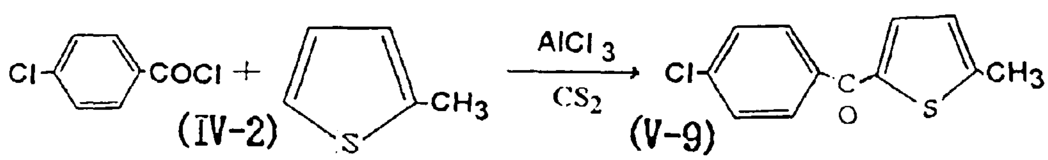


p-Chlorobenzoyl chloride (III-3) (13.1g, 0.075 mole) was used as the starting material and treated with 2-methyl-thiophene (IV-2) (15 g, 0.15 mole) according to the procedure described in example I-1-6 to give compound V-9, as a colourless liquid. Yield, 11.7g (66%).

MS(%),m/z: 236 (100) (M+).
IR(KBr)νmax: 1680 $cm^{-1}$(C=O).
$^1$H-NMR ($CDCl_3$)δ:

2.57 (3H, S, $CH_3$),
6.72 (1H, d, J=3.5 Hz, $C_4$-H),
7.25-7.75 (5H, m, $C_3$-H, phenyl-H).

Preparation of 1-benzyl-3-substituted aryl condensed pyrazoles (IX)

The preparation of the compounds (IX), in which $R_2$ represents -COOR (in which R represents $C_{1\text{-}3}$alkyl), H or halogen, and $R_3$, $Ar_2$ and $Ar_3$ are as defined in formula (A), is shown in Scheme 2.

Scheme 2

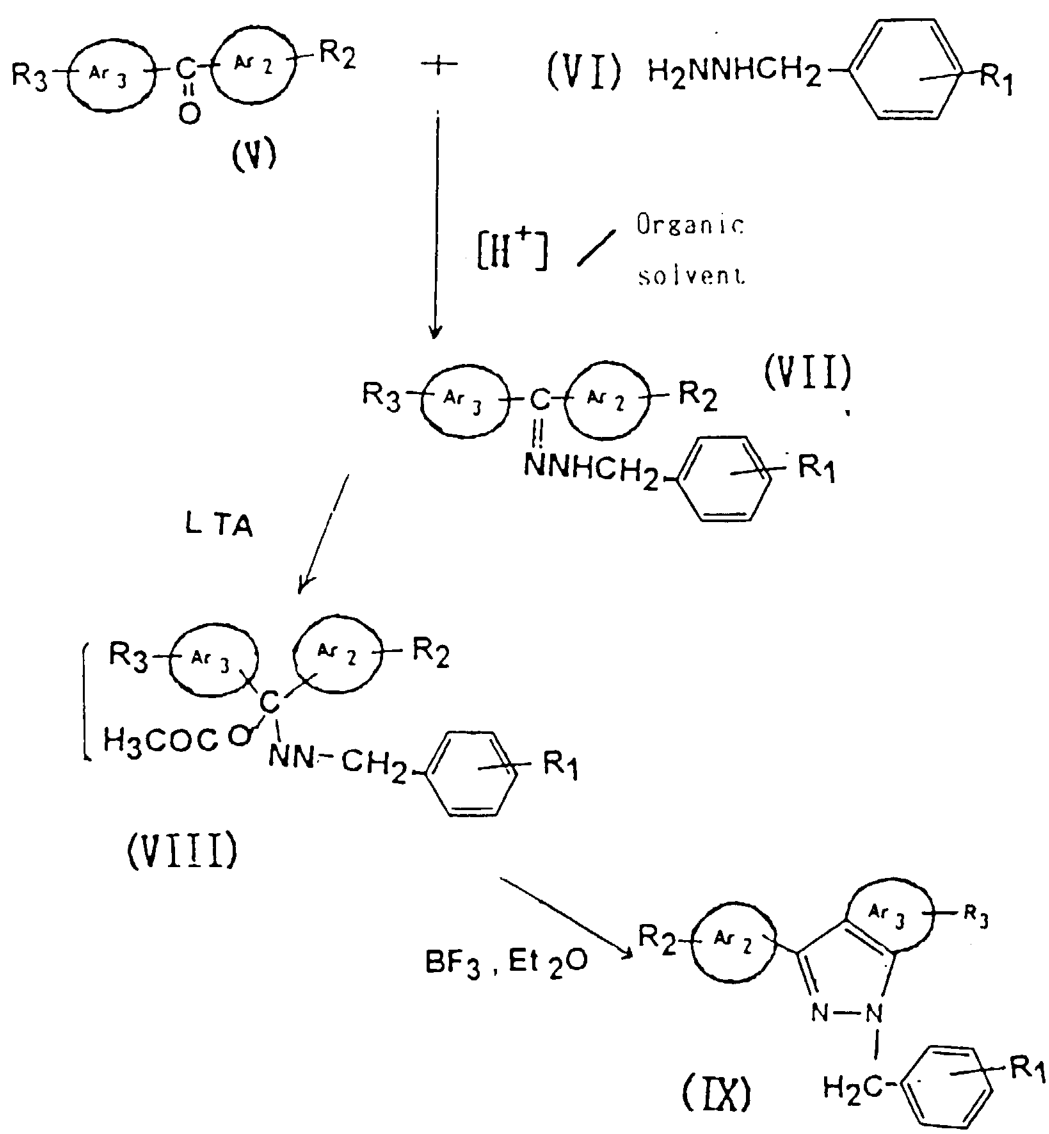


The preparation is performed using the intermediate ketones (V) and substituted benzyl hydrazines (VI) as the starting materials, in organic solvent and Lewis acid under condensed reaction to form the corresponding hydrazones(VII). The residue is purified by column chromatography to give two products, which furthermore convert to each other rapidly to form a mixture. According to S. Yushina et al., (Yakugaku Zasshi vol.97, 955, 1977) these two products were the E form and Z form isomer of hydrazones (VII). Since each of the isomers might undergo the next reaction to

give the same products VIII, no attempt is made to separate the E form and Z form isomer. Thus the hydrazone mixture (VII) is dissolved in a non-polar solvent. While the mixture is stirred vigorously below 40°C, lead tertraacetate and borontrifluoride-etherate mixture are added to oxidize and cyclize the compound. The products are purified by column chromatography, recrystallized to give the corresponding 1-benzyl-3-(substituted aryl)-condensed pyrazoles (IX). The structures are determined using IR, NMR, MS, and elemental analytical data.

EXAMPLE I-2-1

1-Benzyl-1-3-(5''-methoxycarbonylfuryl)-indazole (IX-1)

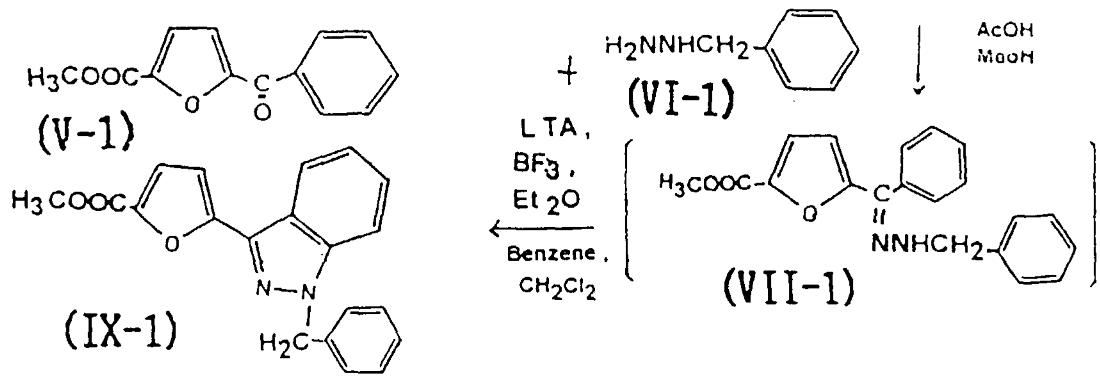


5-Methoxycarbonyl-2-furyl phenyl ketone (V-1) (5.52g, 0.024 mole) was dissolved in methanol (60ml), added to benzylhydrazine (VI-1) (9g, 0.074 mole) and acetic acid (0.5ml) and then heated under refluxing till the reaction was completed. After cooling, the solvent was evaporated and the residue was extracted with chloroform, then washed with dilute HCl solution and water till neutral, then dried over anhydrous magnesium sulfate and filtered. The solvent of the filtrate evaporated to give 5''-methoxycarbonylfuryl phenyl ketone benzylhydrazone (VII-1).

The crude product VII-1 was dissolved in 60ml benzene, then mixed with LTA (28.2g), $BF_3.Et_2O$ (12.2ml), benzene (100ml), and dichloromethane (100ml) under stirring. The mixture was poured into ice-water, the organic layer was washed with water, 10% sodium hydroxide solution till neutral, then dried over anhydrous magnesium sulfate and filtered. The solvent of the filtrate was evaporated and the residue was purified by column chromatography (silica gel-benzene) to give compound IX-1. Yield, 0.8g (10%).

mp: 117-118°C.
MS(%),m/z: 332 (100) (M+).
IR(KBr)νmax: 1720 $cm^{-1}$(C=O).
$^1H$-NMR ($CDCl_3$)δ:

3.95 (3H, S, $CH_3$),
5.66 (2H, S, $-CH_2-$),
7.02 (1H, d, J=3.5 Hz, $C_{3''}$-H),
7.20-7.40 (9H, m, $C_{5,6,7''}$,phenyl-H),
8.26 (1H, d, d, J=8.1, 1.5 Hz, $C_4$-H).

| | | | |
|---|---|---|---|
| Anal (for $C_{20}H_{16}N_2O_3$) Calcd | C, 72.28; | H, 4.85; | N, 8.43. |
| Found | C, 72.50; | H, 4.60; | N, 8.60. |

EXAMPLE I-2-2

1-Benzyl-3-(5''-methoxycarbonyl-2-furyl)-6-methylindazole (IX-2)

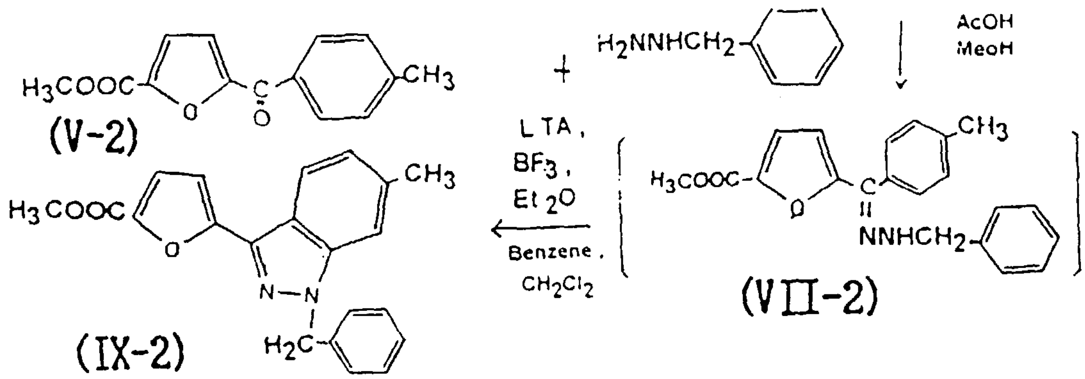


5-Methoxycarbonyl-2-furyl) 4'-methyl phenyl ketone (V-2) (5.85g, 0.024 mole) was used as the starting material and treated, according to the procedure described in example I-2-1 to give compound IX-2. Yield,1.3g (16%).

mp: 102-104°C.
MS(%),m/z: 346 (100) (M+).
IR(KBr)νmax: 1720 $cm^{-1}$(C=O).
$^1$H-NMR (DMSO-$d_6$) δ:

2.46 (3H, S, -$CH_3$),
3.87 (3H, S, -$OCH_3$-),
5.71 (2H, S, -$CH_2$-),
7.14-7.36 (7H, m, $C_{5,3''}$-H,phenyl-H),
7.45 (1H, d, J=3.4 Hz, $C_{4''}$-H),
7.59 (1H, s, $C_7$-H),
8.10 (1H, d, J=8.0 Hz, $C_4$-H).

| | | | |
|---|---|---|---|
| Anal (for $C_{21}H_{18}N_2O_3$) Calcd | C, 72.82; | H, 5.24; | N, 8.09. |
| Found | C, 72.90; | H, 5.21; | N, 8.28. |

EXAMPLE I-2-3

1 -Benzyl-3-(5''-methoxycarbonyl-2-furyl)-6-methoxyindazole (IX-3)

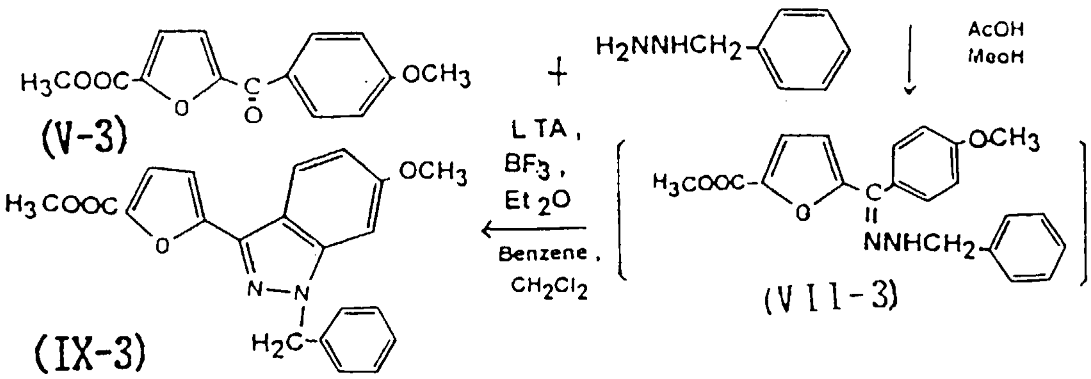

(V-3)
(VII-3)
(IX-3)

5-Methoxycarbonyl-2-furyl 4'-methyl phenyl ketone (V-3) (6.24g, 0.024 mole) was used as the starting material and treated according to the procedure described in example I-2-1 to give compound IX-3. Yield, 1.8g (21%).

mp: 108-109°C.
MS(%),m/z: 362 (100) (M+).
IR(KBr)νmax: 1710 $cm^{-1}$(C=O).
$^1$H-NMR (DMSO-$d_6$)δ:

3.85 (3H, S, $OCH_3$),

3.88 (3H, S, $-\overset{O}{\overset{\|}{C}}-OCH_3-$),

5.71 (2H, S, $-CH_2-$),
6.95 (1H, d, J=8.5 Hz, $C_5$-H),
7.16 (1H, d, J=3.5 Hz, $C_{3''}$-H),
7.24-7.36 (6H, m, $C_7$-H, phenyl-H),
7.40 (1H, d, J=3.5 Hz, $C_{4''}$-H),
7.98 (1H, d, J=8.5 Hz, $C_4$-H).

| | | | |
|---|---|---|---|
| Anal (for $C_{21}H_{18}N_2O_4$) Calcd | C, 69.60; | H, 5.01; | N, 7.73. |
| Found | C, 69.40; | H, 5.21; | N, 7.80. |

EXAMPLE 1-2-4

1-Benzyl-3-(5"-methoxycarbonyl-2-furyl)-6-fluoroindazole (IX-4)

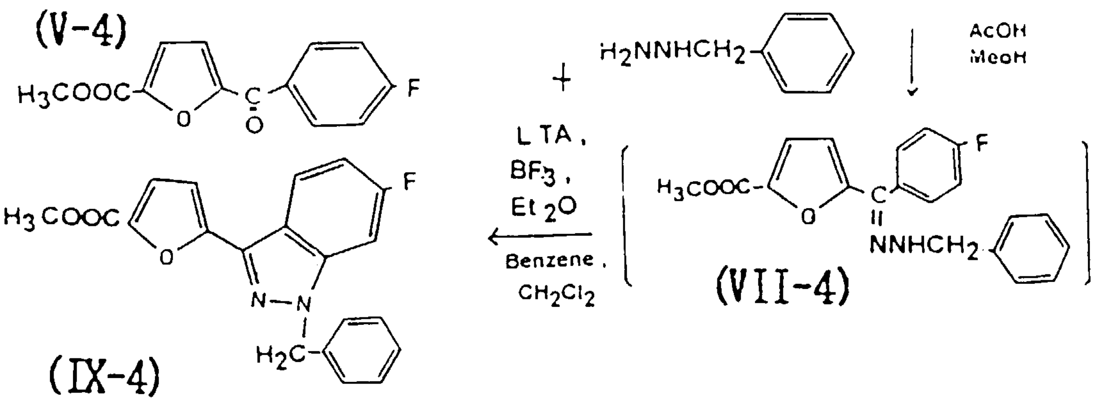


p-Fluorophenyl 5-methoxycarbonyl-2-furyl ketone (V-4) (5.96g, 0.024 mole) was used as the starting material and treated according to the procedure described in example 1-2-1 to give compound IX-4. Yield, 0.4g (4.8%).

mp: 108-109°C.
IR(KBr)νmax: 1710 $cm^{-1}$ (C=O).
1 H-NMR ($DMSO-d_6$) δ:

3.87 (3H, S, $CH_3$),
5.73 (2H, S, $-CH_2-$),
7.18-7.37 (7H, m, $C_{5,3''}$-H,phenyl-H),
7.45 (1H, d, J=3.5 Hz, $C_{4''}$-H),
7.77 (1H, dd, J=10.0, 1.5 Hz, $C_7$-H),
8.17 (1H, d, d, J=8.0, 6.3 Hz, $C_4$-H).

| | | | |
|---|---|---|---|
| Anal (for $C_{20}H_{15}FN_2O_3$) Calcd | C, 68.57; | H, 4.32; | N, 8.00. |
| Found | C, 68.39; | H, 4.40; | N, 7.90. |

EXAMPLE I-2-5

1-Benzyl-3-(5''-methoxycarbonyl-2-furyl) thieno[3,2-c] pyrazole (IX-5)

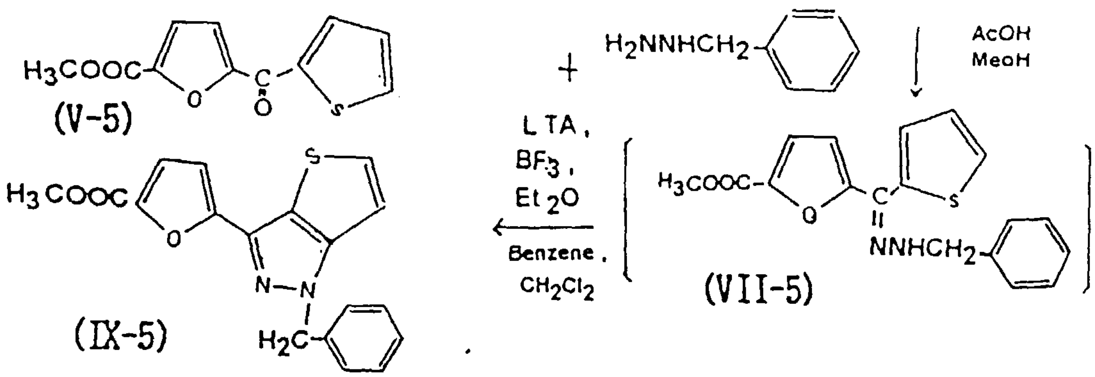


(V-5) (VII-5) (IX-5)

(5-Methoxycarbonyl-2-furyl 2'-thiophenyl ketone (V-5) (5.7g, 0.024 mole) was used as the starting material and treated according to the procedure described in example I-2-1 to give compound IX-5. Yield, 1.2g (14.8%).

mp: 142-143°C.
MS(%),m/z: 338 (100) (M+).
IR(KBr)νmax: 1720 $cm^{-1}$ (C=O).
$^1$H-NMR (DMSO-$d_6$)δ:

3.85 (3H, S, $CH_3$),
5.62 (2H, S, -$CH_2$-),
6.92 (1H, d, J=3.5 Hz, $C_{3''}$-H),
7.24 (1H, d, J=4.8 Hz, $C_6$-H),
7.26-7.35 (5H, m, phenyl-H),
7.43 (1H, d, J=3.5 Hz, $C_{4''}$-H),
7.77 (1H, d, J=4.8, 1.5 Hz, $C_5$-H).

| | | | |
|---|---|---|---|
| Anal (for $C_{18}H_{14}N_2O_3S$: Calcd | 63.89; | H, 4.17; | N, 8.28. |
| Found | C, 63.71; | H, 4.30; | N, 8.50. |

EXAMPLE I-2-6

1-Benzyl-3-phenyl-5-methyl furo[3,2-c]pyrazole (IX-6)

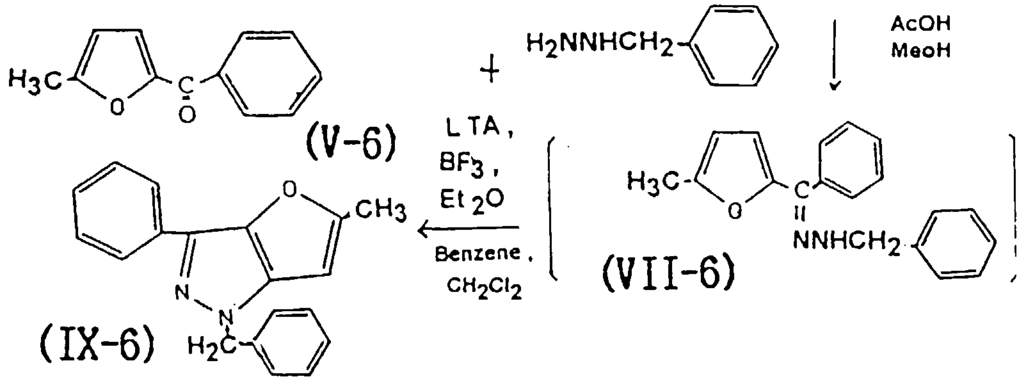


5-Methyl-2-furyl phenyl ketone (V-6) (4.5g, 0.024 mole), was used as the starting material and treated according to the procedure described in example I-2-1 to give compound IX-6. Yield, 2.1g (30%).

mp: 143-145°C.
MS(%),m/z: 288 (100) (M+).
$^1$H-NMR ($CDCl_3$)δ:

2.37 (3H, S, $CH_3$),
5.28 (2H, S, -$CH_2$-),
5.53 (1H, S, $C_5$-H),
7.23-8.17 (10H, m, phenyl-H).

| | | | |
|---|---|---|---|
| Anal (for $C_{19}H_{16}N_2O$) Calcd: | C, 79.14; | H, 5.59; | N, 9.72. |
| Found: | C, 79.32; | H, 5.68; | N, 9.52. |

EXAMPLE I-2-7

1-Benzyl-3-(p-methylphenyl-5-methyl furo[3,2-c]pyrazole (IX-7)

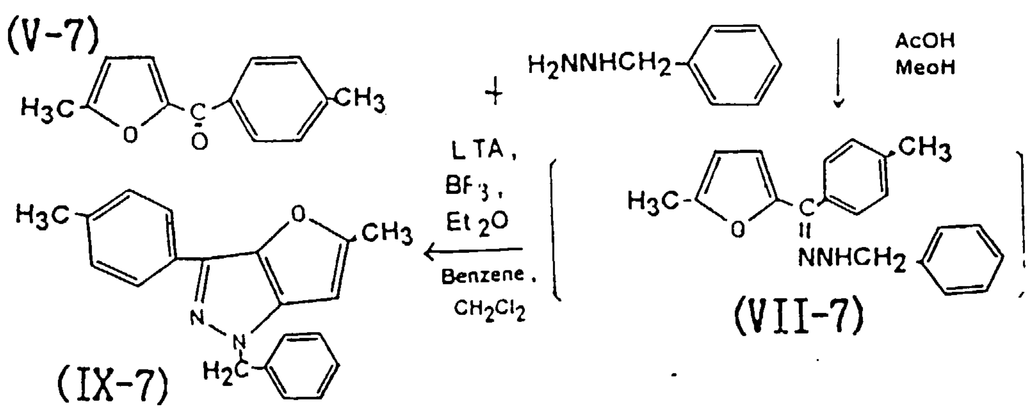

5-Methyl-2-furyl p-methylphenyl ketone (V-7) (4.8g, 0.024 mole) was used as the starting material and treated according to the procedure described in example I-2-1 to give compound IX-7. Yield, 2.3g (32%).

mp: 138-140°C.
MS(%),m/z: 302 (100) (M+).
$^1$H-NMR (CDCl3) δ:

2.30 (3H, S, 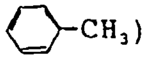),

2.36 (3H, S, 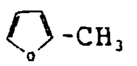 ),

5.28 (2H, S, $-CH_2-$ ),
5.50 (1H, S, $C_5$-H),
7.21-8.10 (9H, m, phenyl-H).

| | | | |
|---|---|---|---|
| Anal (for $C_{20}H_{18}N_2O$) Calcd | C, 79.44; | H, 6.00; | N, 9.27. |
| Found | C, 79.21; | H, 6.21; | N, 9.51. |

EXAMPLE I-2-8

1-Benzyl-3-(p-chlorophenyl)-5-methyl furo[3,2-c]pyrazole (IX-8)

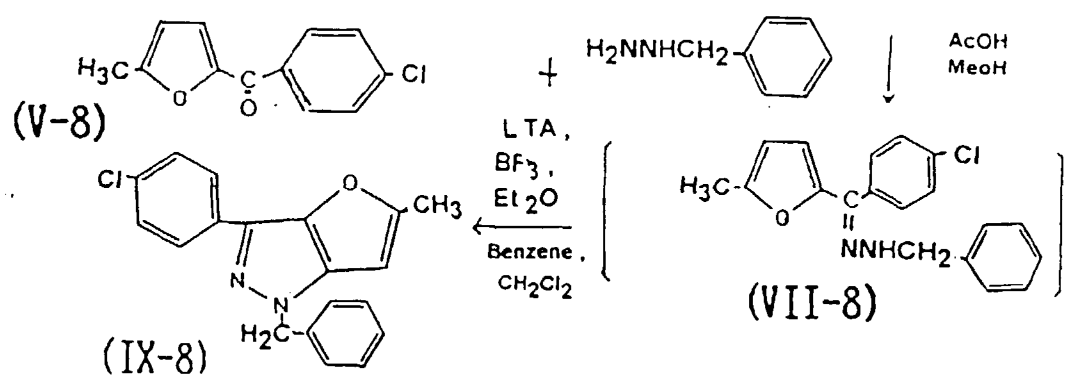

(V-8)
(VII-8)
(IX-8)

p-Chlorophenyl 5-methyl-2-furyl ketone (V-8) (5.3g, 0.024 mole) was used as the starting material and treated according to the procedure described in example I-2-1 to give compound IX-8. Yield, 2.6g (34%).

mp: 151-152%.
MS(%),m/z: 322 (100) (M+).
$^1$H-NMR ($CDCl_3$)δ:

2.34 (3H, S, $CH_3$),
5.34 (2H, S, $-CH_2-$),
6.29 (1H, S, $C_5$-H),
7.43-7.83 (9H, m, phenyl-H).

| | | | |
|---|---|---|---|
| Anal (for $C_{19}H_{15}ClN_2O$) Calcd | C, 70.70; | H, 4.68; | N, 8.68. |
| Found | C, 70.83; | H, 4.52: | N, 8.78. |

EXAMPLE I-2-9

1-Benzyl-3-(p-chlorophenyl)-5-methyl thieno[3,2-c]pyrazole (IX-9)

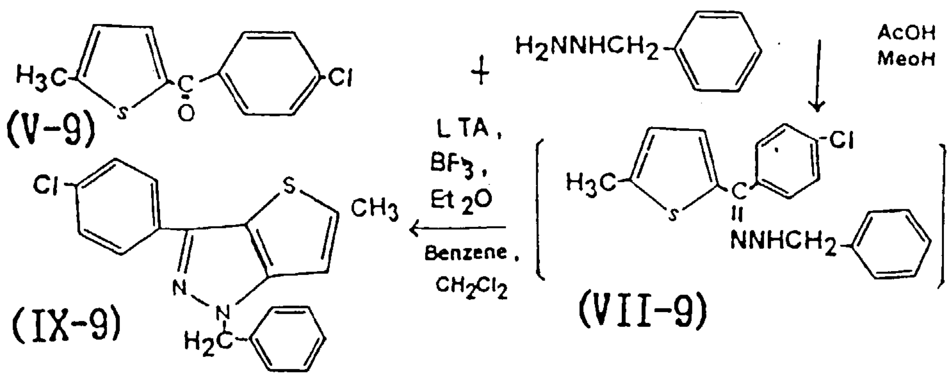


p-Chlorophenyl 5-methyl-2-thienyl ketone (V-9) (5.7g, 0.024 mole) was used as the starting materials and treated according to the procedure described in example I-2-1 to give compound IX-9. Yield, 2.5g (31%).

mp: 115-117°C.
MS(%),m/z: 338 (100) (M+).
$^1$H-NMR ($CDCl_3$)δ:

2.47 (3H, S, $CH_3$),
5.43 (2H, S, $-CH_2-$),
6.35 (1H, S, $C_5$-H),
7.24-7.79 (9H, m, phenyl-H).

| | | | |
|---|---|---|---|
| Anal (for $C_{19}H_{15}ClN_2S$) Calcd | C, 67.35; | H, 4.46; | N, 8.27. |
| Found | C, 67.53; | H, 4.21; | N, 8.20. |

EXAMPLE I-2-10

1-(p-Methylbenzyl)-3-phenyl-5-methyl furo[3,2-c] pyrazole (IX-10)

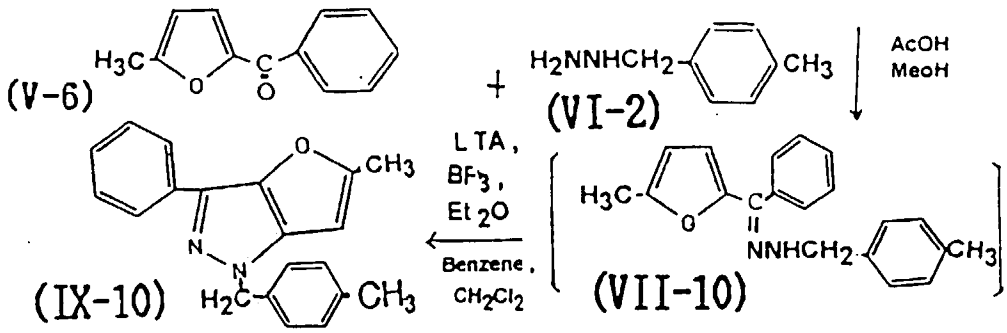

(V-6) (VI-2) (VII-10) (IX-10)

5-Methyl-2-furyl phenyl ketone (V-6) (4.5g, 0.024 mole) and p-methylbenzyl hydrazine (VI-2) (10g, 0.074 mole) were used as the starting materials and treated according to the procedure described in example I-2-1 to give compound IX-10. Yield, 2.5g (35%).

mp: 130-132°C.
MS(%),m/z: 302 (100) (M+).
$^1$H-NMR (CDCl3) δ:

2.31 (3H, S, 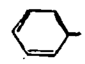$-CH_3$),

2.35 (3H, S, 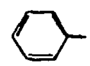$-CH_3$),

5.26 (2H, S, $-CH_2-$),
5.52 (1H, S, $C_5$-H),
7.20-8.00 (9H, m, phenyl-H).

| | | | |
|---|---|---|---|
| Anal (for $C_{20}H_{18}N_2O$) Calcd | C, 79.44; | H, 6.00; | N, 9.26. |
| Found | C, 79.62; | H, 6.30; | N, 9.50. |

EXAMPLE I-2-11

1-(p-Chlorobenzyl)-3-phenyl-5-methyl furo[3,2-c] pyrazole (IX-11)

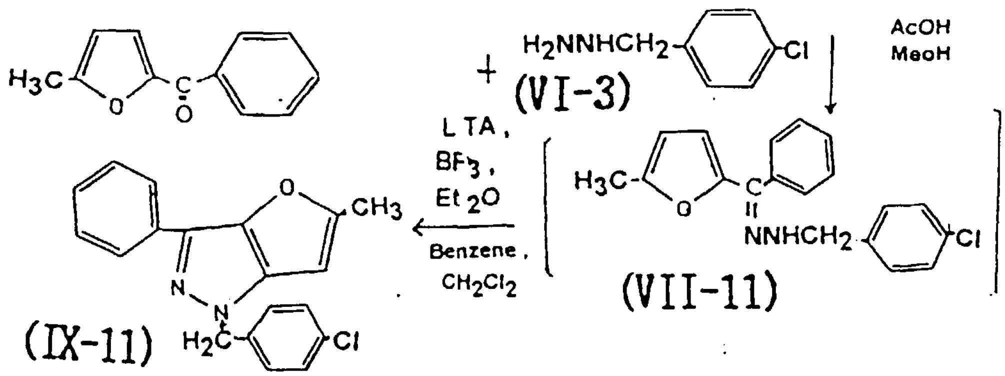


5-Methyl-2-furyl phenyl ketone (V-6) (4.5g, 0.024 mole) and p-chlorobenzyl hydrazine (VI-3) (11.4 g, 0.073 mole) were used as the starting material and treated according to the procedure described in example I-2-1 to give compound IX-11. Yield, 2.3g (30%).

MS(%),m/z: 322 (100) (M+).
$^1$H-NMR ($CDCl_3$)δ:

2.36 (3H, S, $CH_3$),
5.26 (2H, S, $-CH_2-$),
5.53 (1H, S, $C_5$-H),
7.10-8.10 (9H, m, phenyl-H);

| | | | |
|---|---|---|---|
| Anal (for $C_{19}H_{15}ClN_2O$) Calcd | C, 70.70; | H, 4.68; | N, 8.68. |
| Found: | C, 70.90; | H, 4.78; | N, 8.51. |

EXAMPLE I-2-12

1-(p-Methoxybenzyl)-3-phenyl-5-methl furo[3,2-c]pyrazole (IX-12)

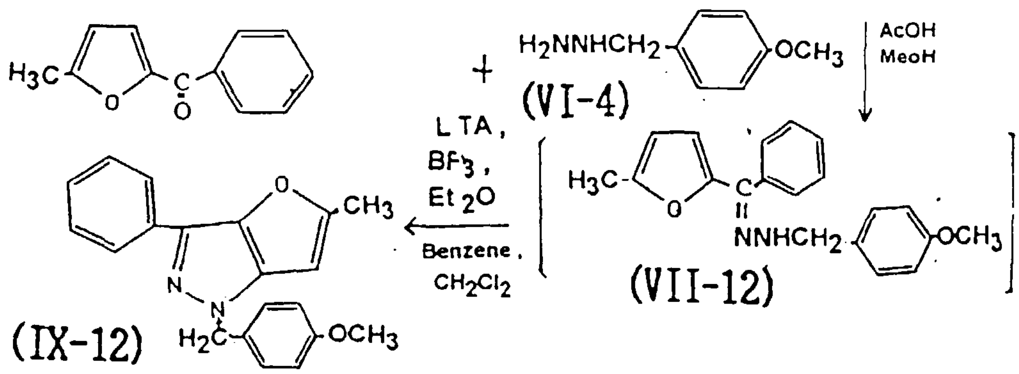


(VI-4) (VII-12) (IX-12)

p-Methoxy benzyl hydrazine (VI-4) (11.1g, 0.073 mole) and 5-methyl-2-furyl phenyl ketone (V-6) (4.5g, 0.024 mole) were used as the starting material and treated according to the procedure described in example I-2-1 to give compound IX-12. Yield, 2.2g (29%).

mp: 130-132°C.
MS(%),m/z: 318 (100) (M+).
$^1$H-NMR ($CDCl_3$)δ:

2.35 (3H, S, $C_4$-$CH_3$),
5.26 (2H, S, -$CH_2$-),
5.55 (1H, S, $C_5$-H),
6.80 (2H, d, J=8.8 Hz, $C_{3',5'}$-H),
7.11-8.10 (7H, m, phenyl-H).

| | | | |
|---|---|---|---|
| Anal (for $C_{20}H_{18}N_2O_2$) Calcd | C, 75.45; | H, 5.70; | N, 8.80. |
| Found | C, 75.60; | H, 5.51; | N, 8.92. |

EXAMPLE I-2-13

1-Benzyl-3-(p-ethoxycarbonylphenyl)indazole(IX-13)

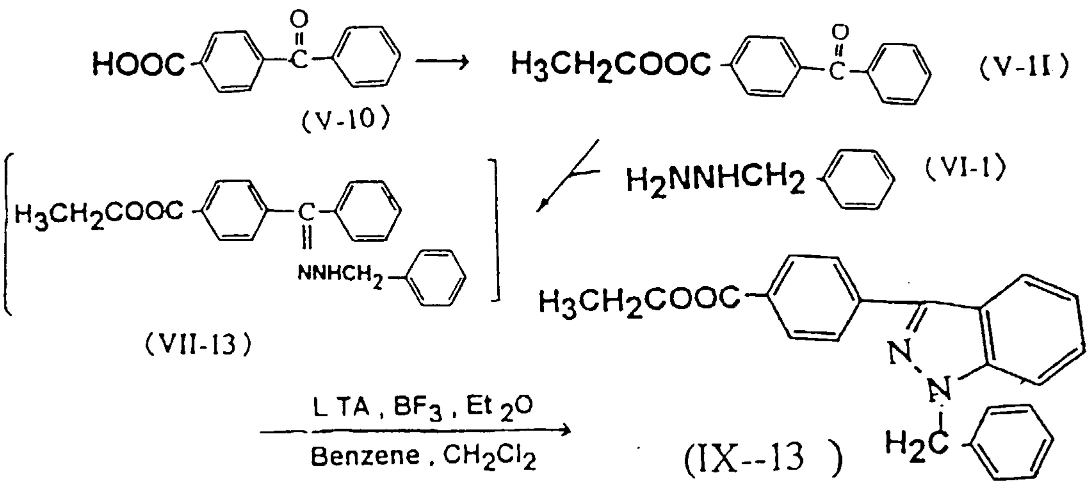

(V-10) (V-11) (VI-1) (VII-13) (IX--13)

p-Benzoylbenzoic acid (V-10) was reported by Werthein E (J Am Chem Soc, Vol 55, 2540, 1933).

Compound V-10(22.6g 0.1 mole) was dissolved in toluene (100 mole). To the solution was added p-toluensulfonic acid (3.0g) and ethanol (15ml) and was boiled under reflux for 24 hours. The reaction mixture was poured into ice water. The organic layer was washed with 5% $NaHCO_3$ solution and then with water, dried over anhydrous magnesium sulfate and filtered. The solvent of filtrate was evaporated and the residue was purified by chromatography on silica gel. Elution with benzene, yielded ethyl p-benzoylbenzoate (V-11) (23.0g, 90%)

Compound V-11 (23.0g, 0.09 mole) and benzyl hydrazine (VI-1) (36.0g, 0.3 mole) was used as the starting material and treated according to the procedure described in example 1-2-1 to give compound IX-13. Yield, 9.6g (30%)

mp: 95-96°C.
IR(KBr)νmax: 1700 $cm^{-1}$(C=O)
MS(%),m/z: 356 (100) (M+).
$^1H$-NMR ($CDCl_3$)δ:

1.35 (3H, t, J=8.0Hz, $-CH_2-\underline{CH_3}$),
4.35 (2H, q, J=8.0Hz, $-CH_2-\underline{CH_3}$),

5.78 (2H, S, $-CH_2-$ 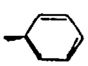 ),

7.40-8.40(13H, m, aromatic protons)

| | | | |
|---|---|---|---|
| Anal (for $C_{23}H_{20}N_2O_2$) Calcd | C, 77.51; | H, 5.66; | N, 7.86. |
| Found | C, 77.30; | H, 5.71; | N, 7.68. |

Preparation of 1-benzyl-3-(hydroxycarbonylaryl) condensed pyrazoles) (X) and 1-benzyl-3-(hydroxymethylaryl) condensed pyrazoles (XI)

The preparation of the compounds (X) and (XI), in which $R_1$,$R_3$,Ar2 and Ar3 are as defined in formula (A), is shown in Scheme 3.

Scheme 3

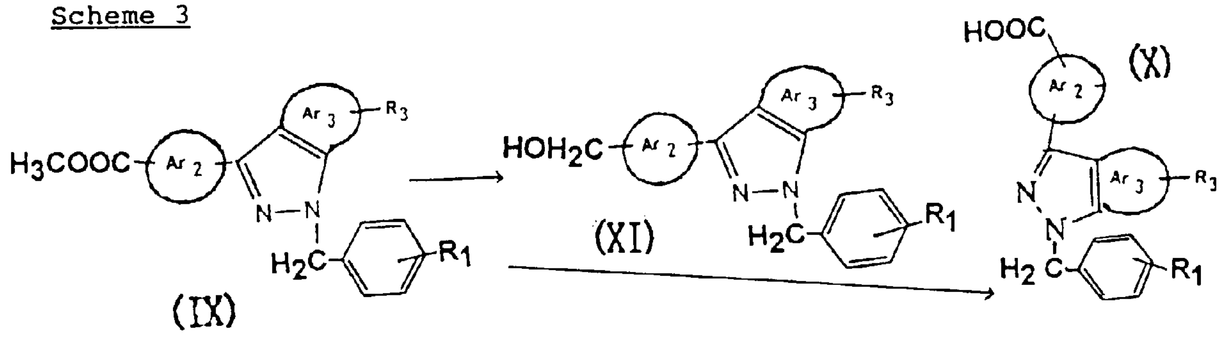

(IX) (XI) (X)

The preparation is performed by hydrolyzing the ester groups of condensed pyrazoles (IX) with acids or bases to form the corresponding carboxylic acids (X). The methyl ester groups of condensed pyrazoles (IX) are reduced with strong reducing agents, eg $LiAlH_4$ or $CaBH_4$ to the corresponding alcohols (XI).

EXAMPLE I-3-1

1-Benzyl-3-(5"-hydroxycarbonyl-2-furyl)-indazole (X-1)

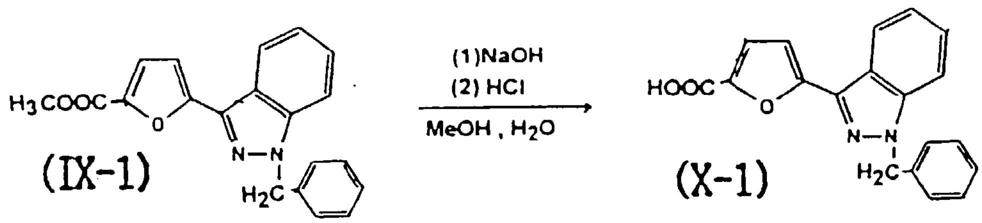


(IX-1) (X-1)

1-Benzyl-3-(5"-methoxycarbonylfuryl)-indazole (IX-1) (100mg, 0.032 mole), was dissolved in a mixture of methanol (8ml) and sodium hydroxide (7.5mg) solution, then heated under refluxing. After cooling, the solvent was evaporated. The residue was dissolved in water (1.5ml), then acidified with diluted HCl solution. The crystals were collected, then recrystallized from acetone to give compound X-1. Yield, 73mg (76.5%).

mp: 202-203°C.
MS(%),m/z: 318 (100) (M+).
IR(KBr)vmax: 3450 $cm^{-1}$(-OH), 1700 $cm^{-1}$(C=O).
$^1$H-NMR (DMSO-$d_6$) δ:

5.76 (2H, S, -$CH_2$),
7.20 (1H, d, J=3.5 Hz, $C_{3''}$-H),
7.26-7.35 (6H, m, $C_5$-H, phenyl-H),
7.38 (1H, d, J=3.5 Hz, $C_{4''}$-H),
7.49 (1H, t, J=8.2 Hz, $C_6$-H),
7.80 (1H, d, J=8.2, 1.5 Hz, $C_7$-H),
8.15 (1H, d, J=8.1, 1.5 Hz, $C_4$-H).

| | | | |
|---|---|---|---|
| Anal (for $C_{19}H_{14}N_2O_3$) Calcd | C, 71.69; | H, 4.43; | N, 8.80. |
| Found | C, 71.91; | H, 4.62; | N, 8.62. |

EXAMPLE I-3-2

1-Benzyl-3-(5"-hydroxycarbonylfuryl-6-methylindazole (X-2)

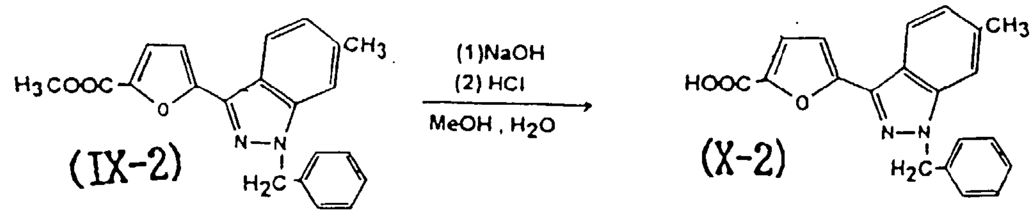


1-Benzyl-3-(5"-methoxycarbonylfuryl)-6-methylindazole (IX-2) (111mg, 0.032 mole) was used as the starting material, and treated according to the procedure described in example I-3-1 to give compound X-2. Yield, 95mg (89%).

mp: 201-202°C.
MS(%),m/z: 332 (100) (M+).
IR(KBr)νmax : 3450 $cm^{-1}$(-0H), 1700 $cm^{-1}$(C=O).
$^1$H-NMR (DMSO-$d_6$)δ:

2.46 (3H, S, -$CH_3$),
5.70 (2H, S, -$CH_2$-),
7.16 (1H, d, J=3.5 Hz, $C_{3''}$-H),
7.23-7.33 (6H, m, $C_5$-H, phenyl-H),
7.38 (1H, d, J=3.5 Hz, $C_{4''}$-H),
7.61 (1H, d, J=1.4 Hz, $C_7$-H),
8.00 (1H, d, J=8.2 Hz, $C_4$-H).

| | | | |
|---|---|---|---|
| Anal (for $C_{20}H_{16}N_2O_3$) Calcd | C, 72.28; | H, 4.85; | N, 8.43. |
| Found | C, 72.51; | | N, 8.23. |

EXAMPLE I-3-3

1-Benzyl-3-(5"-hydroxycarbonylfuryl)-6-methoxyindazole (X-3)

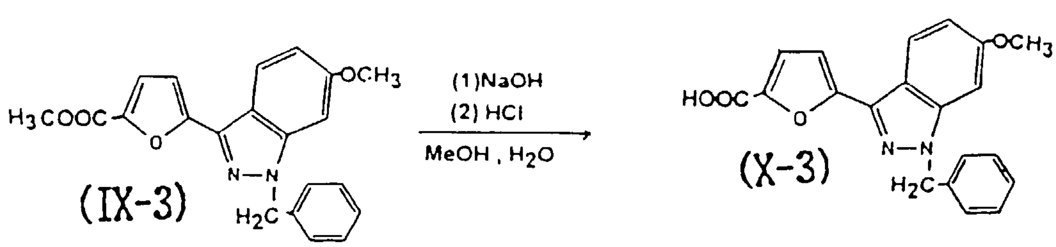


1-Benzyl-3-(5"-methoxycarbonylfuryl)-6-methoxyindazole (IX-3) (116mg, 0.032 mole) was used as the starting material, and treated according to the procedure described in example I-3-1 to give product X-3. Yield, 86.1mg (77.3%).

mp: 222-223°C.

MS(%),m/z: 348 (100) (M+).
IR(KBr)νmax: 3450 $cm^{-1}$(-OH), 1710 $cm^{-1}$(C=O).
$^1$H-NMR (DMSO-$d_6$)δ:

3.84 (3H, S, $CH_3$),
5.70 (2H, S, -$CH_2$-),
6.95 (1H, dd, J=8.3, 1.8 Hz, $C_5$-H),
7.12 (1H, d, J=3.4 Hz, $C_{3''}$-H),
7.25-7.38 (7H, m, $C_{7,4''}$-H, phenyl-H),
7.98 (1H, d, J=8.3 Hz, $C_4$-H);

| | | | |
|---|---|---|---|
| Anal (for $C_{20}H_{16}N_2O_4$) Calcd | C, 68.96; | H, 4.63; | N, 8.04. |
| Found | C, 68.71; | H, 4.39; | N, 8.23. |

EXAMPLE I-3-4

1-Benzyl-3-(5"-hydroxycarbonylfuryl-6-fluoroindazole (X-4)

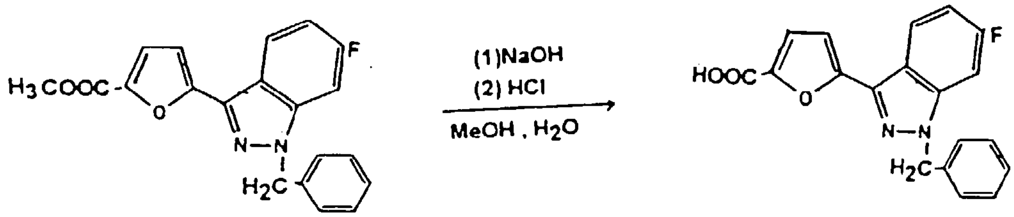


1-Benzyl-3-(5"-methoxycarbonylfuryl)-6-fluoroindazole (IX-4) (112mg, 0.032 mole) was used as the starting material, and treated according to the example procedure described in I-3-1 to give compound X-4. Yield, 70mg (65%).

mp: 252-253°C.
MS(%),m/z: 336 (100) (M+).
IR(KBr)νmax: 3450 $cm^{-1}$(-OH), 1690 $cm^{-1}$(C=O).
$^1$H-NMR (DMSO-$d_6$)δ:

5.72 (2H, S, -$CH_2$-),
7.21 (1H, d, J=3.5 Hz, $C_{3''}$-H),
7.23-7.33 (6H, m, $C_5$-H, phenyl-H),
7.39 (1H, d, J=3.5 Hz, $C_{4''}$-H),
7.79 (1H, dd, J=9.8, 1.8 Hz, $C_7$-H),
8.17 (1H, dd, J=8.5, 5.3 Hz, $C_4$-H).

| | | | |
|---|---|---|---|
| Anal (for $C_{19}H_{13}FN_2O_3$) Calcd | C, 67.86; | H, 3.90; | N, 8.33. |
| Found | C, 67.97; | H, 3.82; | N, 8.31. |

EXAMPLE I-3-5

1-Benzyl-3-(5"-hydroxycarbonylfuryl)-thieno[3,2-c]pyrazole(X-5)

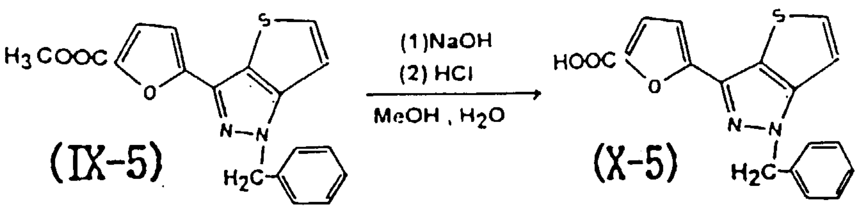


(IX-5) (X-5)

1-Benzyl-3-(5"-methoxycarbonylfuryl) thieno[3,2-c]pyrazole (IX-5) (108mg, 0.032 mole) was used as the starting material and treated according to the procedure described in example I-3-1 to give compound X-5. Yield, 83.3mg (80.3%).

mp: 221-224°C.
MS(%),m/z: 324 (100) (M+).
IR(KBr)vmax: 3500 $cm^{-1}$(-OH), 1720 $cm^{-1}$(C=O).
$^1$H-NMR (DMSO-$d_6$)δ:

5.62 (2H, S, -$CH_2$-),
6.90 (1H, d, J=3.5 Hz, $C_{3''}$-H),
7.26 (1H, d, J=4.8 Hz, $C_6$-H),
7.25-7.35 (6H, m, $C_{4''}$-H, phenyl-H),
7.78 (1H, d, J=4.8 Hz, $C_5$-H).

| | | | |
|---|---|---|---|
| Anal (for $C_{17}H_{12}N_2O_3$) Calcd | C, 62.95; | H, 3.73; | N, 8.64. |
| Found | C, 62.70; | H, 3.51; | N, 8.80. |

EXAMPLE I-3-6

1-Benzyl-3-(5"-hydroxycarbonylfuryl)-indazole (X-6)

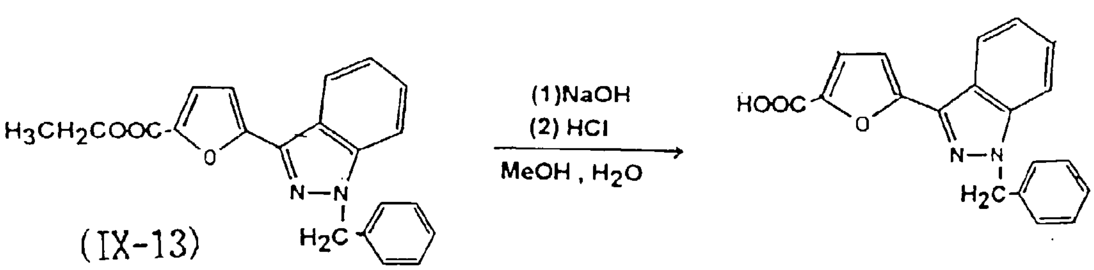


(IX-13)

1-Benzyl-3-(p-ethoxycarbonylphenyl)indazole (1X-13) (14g, 0.04 mole) was used as the starting material and treated according to the procedure described in example I-3-1 to give compound X-6. Yield, 9.6g (75%).

mp: 204-205°C. (d.)
MS(%),m/z: 328 (100) (M+).

IR(KBr)νmax: 3500-3300cm$^{-1}$(-OH), 1710 cm$^{-1}$(C=O).
$^1$H-NMR (DMSO-$d_6$)δ:

5.77 (2H, S, $-CH_2$- 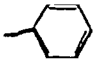 ),

7.28-8.18(13H. m, aromatic prorons)

| | | | |
|---|---|---|---|
| Anal (for $C_{21}H_{16}N_2O_2$) Calcd | C, 76.81; | H, 4.91; | N, 8.53. |
| Found | C, 76.98; | H, 4.83; | N, 8.75. |

EXAMPLE 1-3-7

1-Benzyl-3-(5"-hydroxymethylfuryl)-indazole (XI-1)

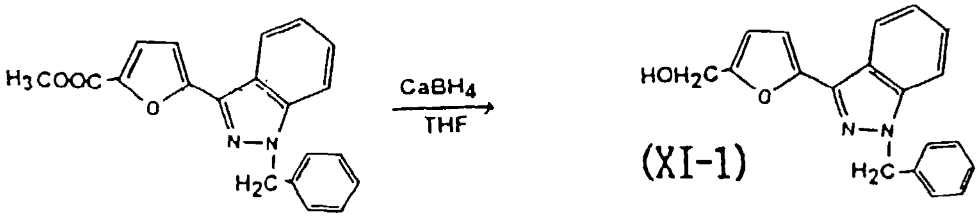

(XI-1)

1-Benzyl-3-(5"-methoxycarbonylfuryl)-indazole (IX-1) (88g, 0.027 mole) was dissolved in a homogenous solution of THF (30 ml) dispersed with calcium borohydride (56 mg, 0.08 mmole). The mixture was heated under refluxing and then filtered. The solvent was evaporated and the residue was recrystallized from n-hexane and the purified by column chromatography (silica gel-n-hexane: ethyl acetate) to give compound XI-1. Yield, 70mg (87%).

mp: 108-109°C.
MS(%),m/z: 304 (100) (M+).
IR(KBr)νmax: 3350 cm$^{-1}$(-0H).
$^1$H-NMR (DMSO-$d_6$)δ:

4.51 (2H, d, J=5.5 Hz, $-CH_2$-O-),
5.31 (1H, t, J=5.5 Hz, -OH),

5.70 (2H, S, $-CH_2$- 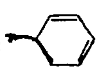 ),

6.47 (1H, d, J=3.4 Hz, $C_{4''}$-H),
6.95 (1H, d, J=3.4 Hz, $C_{3''}$-H),
7.20-7.35 (6H, m, $C_5$-H, phenyl-H),
7.44 (1H, t, J=8.2 Hz, $C_6$-H),
7.73 (1H, dd, J=8.2, 1.8 Hz, $C_7$-H),
8.11 (1H, dd, J=8.2, 1.0 Hz, $C_4$-H).

| | | | |
|---|---|---|---|
| Anal (for $C_{19}H_{16}N_2O_2$) Calcd | C, 74.98; | H, 5.30; | N, 9.20. |
| Found | C, 74.76; | H, 5.61; | N, 9.31. |

EXAMPLE I-3-8

1-Benzyl-3-(5"-hydroxymethylfurl)-6-methylindazole (XI-2)

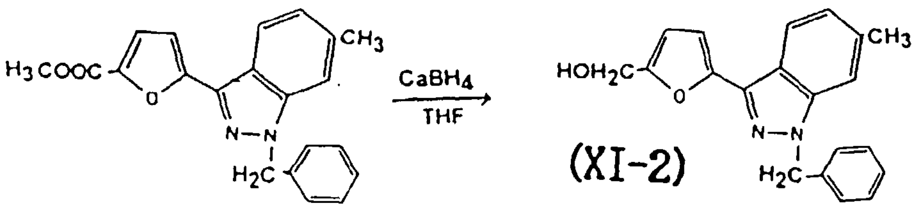


(XI-2)

1-Benzyl-3-(5"-methoxycarbonylfuryl)-6-methylindazole (IX-2) (92mg, 0.027 mole) was used as the starting material and treated according to the procedure described in example 1-3-6 to give compound XI-2. Yield, 74mg (88%).

mp: 112-114°C.
MS(%),m/z: 318 (100) (M+).
IR(KBr)vmax: 3400 $cm^{-1}$(-OH).
$^1H$-NMR (DMSO-$d_6$)δ:

2.44 (3H, S, $CH_3$),
4.50 (2H, d, J=5.2 Hz, $-CH_2-O-$),
5.30 (1H, br, -OH),

5.64 (2H, S, $-CH_2$- 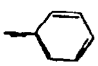 ),

6.45 (1H, d, J=3.3 Hz, $C_{4''}$-H),
6.92 (1H, d, J=3.3 Hz, $C_{3''}$-H),
7.08 (1H, dd, J=8.3, 1.0 Hz, $C_5$-H),
7.19-7.36 (5H, m, phenyl-H),
7.52 (1H, d, J=1.0 Hz, $C_7$-H),
7.98 (1H, dd, J=8.3, 1.0 Hz, $C_4$-H).

| | | | |
|---|---|---|---|
| Anal (for $C_{20}H_{18}NO_2$) Calcd | C, 75.45; | H, 5.70; | N, 8.80. |
| Found | C, 71.50; | H, 5.52; | N, 8.09. |

EXAMPLE 1-3-9

1-Benzyl-3-(5"-hydroxymethylfuryl)-6-methoxylindazole (XI-3)

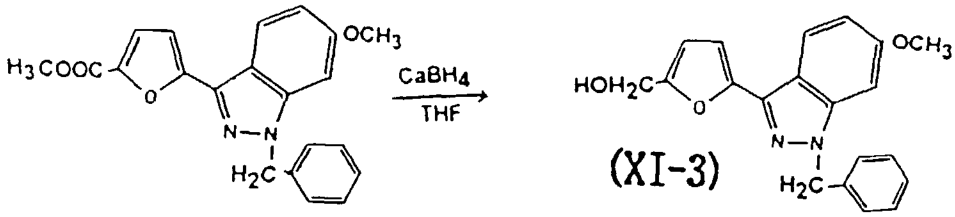


(XI-3)

1-Benzyl-3-(5"-methoxycarbonylfuryl)-6-methoxyindazole (IX-3) (96mg, 0.027 mole) was used as the starting material and treated according to the procedure described in example I-3-6 to give compound XI-3. Yield, 80mg (90%).

mp: 109-110°C.
MS(%),m/z: 334 (100) (M+).
IR(KBr)νmax: 3300íπ3400 $cm^{-1}$(-OH).
$^1H$-NMR ($CDCl_3$)δ:

1.90 (1H, br, OH),
3.80 (3H, S, $-CH_3$),
4.74 (2H, d, J=4.9 Hz, $-CH_2-O-$),
5.59 (2H, S, $-CH_2-$ 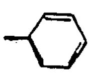 ),
6.47 (1H, d, J=3.2 Hz, $C_{4''}$-H),
6.59 (1H, d, J=2.0 Hz, $C_7$-H),
6.84 (1H, d, J=3.2, 1.0 Hz, $C_{3''}$-H),
6.88 (1H, dd, J=8.5, 1.5 Hz, $C_5$-H),
7.17-7.31 (5H, m, phenyl-H),
7.91 (1H, d, J=8.5 Hz, $C_4$-H).

| | | | |
|---|---|---|---|
| Anal (for $C_{20}H_{18}N_2O_3$) Calcd | C, 71.84; | H, 5.43; | N, 8.38. |
| Found | C, 71.65; | H, 5.25; | N, 8.51. |

EXAMPLE I-3-10

1-Benzyl-3-(5''-hydroxymethylfuryl)-6-fluoroindazole (XI-4)

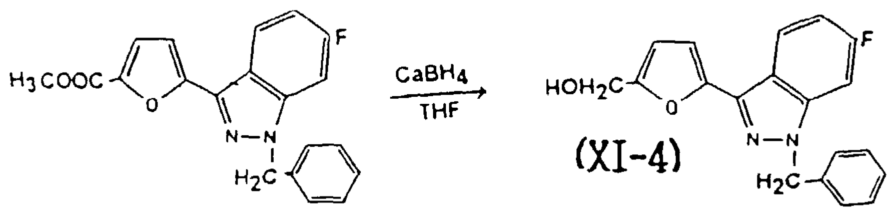


(XI-4)

1-Benzyl-3-(5''-methoxycarbonylfuryl)-6-fluoroindazole (IX-4) (93mg, 0.027 mole) was used as the starting material and treated according to the procedure described in example I-3-6 to give compound XI-4. Yield, 75mg (88%).

mp: 110-112°C.
MS(%),m/z: 322 (100) (M+).
IR(KBr)νmax: 3450 $cm^{-1}$(-OH).
$^1H$-NMR (DMSO-$d_6$)δ:

4.49 (2H, br, $-CH_2$-O-),
5.45 (1H, br, -OH),
5.88 (1H, S, $-CH_2-$ 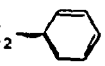 ),
6.48 (1H, d, J=3.2 Hz, $C_{4''}$-H),
6.98 (1H, d, J=3.2 Hz, $C_{3''}$-H),
7.10-7.18 (1H, m, $C_5$-H),
7.24-7.36 (5H, m, phenyl-H),
7.70 (1H, dd, J=10.0, 2.0 Hz, $C_7$-H),
8.15 (1H, dd, J=8.5, 5.1 Hz, $C_4$-H).

| | | | |
|---|---|---|---|
| Anal (for $C_{19}H_{15}FN_2O_2$) Calcd | C, 70.80; | H, 4.69; | N, 8.69. |
| Found | C, 70.59; | H, 4.41; | N, 8.41. |

EXAMPLE I-3-11

1-Benzyl-3-(5''-hydroxymethylfuryl)-thieno[3,2-c]pyrazole (XI-5)

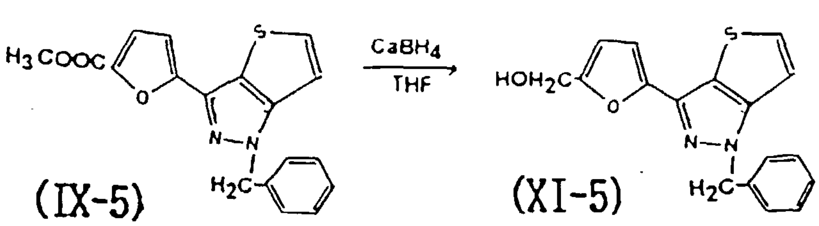


(IX-5) (XI-5)

1-Benzyl-3-(5''-methoxycarbonylfuryl)thieno[3,2-c]pyrazole(IX-5) (90mg, 0.027 mole) was used as the starting material and treated according to the procedure described in example I-3-6 to give compound XI-5. Yield, 63.4mg (76%).

mp: 103-104°C.
MS(%),m/z: 310 (100) (M+).
IR(KBr)νmax: 3360 $cm^{-1}$(-OH).
$^1H$-NMR (DMSO-$d_6$)δ:

4.46 (2H, d, J=5.3 Hz, -$CH_2$-O-),
5.27 (1H, t, J=5.3 Hz, -OH),

5.55 (2H, S, -$CH_2$-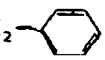 ),

6.43 (1H, d, J=3.2 Hz, $C_{4''}$-H),
6.64 (1H, d, J=3.2 Hz, $C_{3''}$-H),
7.20 (1H, d, J=4.8 Hz, $C_6$-H),
7.27-7.35 (5H, m, phenyl-H),
7.72 (1H, d, J=4.8 Hz, $C_5$-H).

| | | | |
|---|---|---|---|
| Anal (for $C_{17}H_{14}N_2O_2S$) Calcd | C, 65.79; | H, 4.55; | N, 9.03; |
| Found | C, 65.58; | H, 4.70; | N, 9.31. |

EXAMPLE I-3-12

1-Benzyl-3-(p-hydroxymethylphenyl)indazole (XI-6)

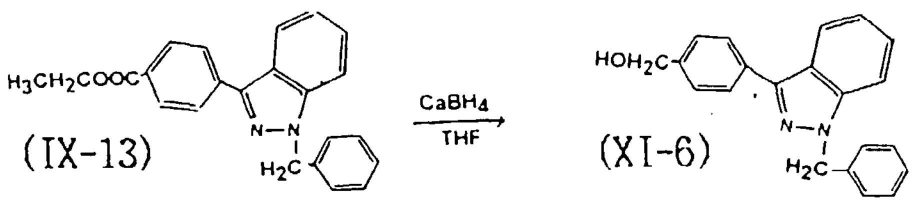


(IX-13) (XI-6)

1-Benzyl-3-(p-ethoxycarbonylphenyl)indazole (IX-13) (9.6g, 0.027 mole) was used as the starting material and treated according to the procedure described in example I-3-6 to give compound XI-6. Yield, 6.4mg (81%).

mp: 110-112°C.
MS(%),m/z: 314 (100) (M+).
IR(KBr)νmax: 3300-2500 $cm^{-1}$(-OH).
$^1H$-NMR (DMSO-$d_6$)δ:

4.58 (2H, d, J=5.2 Hz, -$CH_2$-O-),
5.31 (1H, t, J=5.2 Hz, OH),

5.73 (2H, S, -$CH_2$-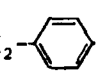 ),

7.23-8.17 (13H, m, aromatic protons.

| | | | |
|---|---|---|---|
| Anal (for $C_{21}H_{18}N_2O$) Calcd | C, 80.23; | H, 5.77; | N, 8.91; |
| Found | C, 80.45; | H, 5.62; | N, 8.99. |

EXAMPLE A

A typical tablet which may be prepared by conventional tabletting techniques contains

| | |
|---|---|
| active compound | 40 mg |
| lactose | 30 mg |
| starch | 8 mg |
| magnesium stearate | 10 mg |
| corn starch | 12 mg |

EXAMPLE B

Inhibiting activity on platelet aggregation

(A) Preparation of aggregation inducing agent.

1. Collagen (bovine tendon) in 15 mM aqueous acetic acid was ground at 4°C to form a well dispersed suspension, and dispensed in 1mg/ml and stocked at -70°C. Before use, it was thawed and well ground.
2. PAF was dissolved in $CCl_4$ and stocked at 20°C. Before use, it was diluted with deionized water.
3. Adenosine (ADP) and sodium arachidonate (AA) were dissolved in deionized water for use.

(B) Preparation of platelets.

A suspension of platelets was prepared according to the reported method. 100 mM of EDTA and the blood from rabbit's ear was mixed in the ratio of 1:4, and immediately separated by centrifuge (120 X g) at room temperature for 10 minutes. The platelets-enriched upper layer plasma was subjected to centrifugation (500 X g) for 10 minutes. After the plasma was removed, the platelets in lower layer were suspended in the Tyrode solution containing EDTA (2mM) and bovine serum protein (3.5mg/ml), and subjected to centrifugation (500 X g) again for 10 minutes. The platelets obtained were suspended in a Tyrode solution containing no EDTA, and adjusted to about 4.5x10 cell/ml by a counter. 1 mM of calcium ion ($Ca^{2+}$) was added to the suspension. 30 minutes after the addition, the experiment was conducted. The composition, of Tyrode: bovine serum protein, NaCl (136.9), KCl (2.7), $Na_3PO_3$ (0.4), $NaHCO_3$ (11.9), glucose (11.1).

(C) Platelet aggregation and ATP release reaction test.

The method reported by G.V.R.Born (J Physiol, Vol 168, 178, 1963) was used to determine the platelet aggregation, in which a Lumi aggregation meter (Model 1020, Payton, Canada) was used. 0.4ml platelet suspension was added into a little glass tube coated with silicone, and stirred at 900 rpm with a small magnetic stirrer. Unless otherwise specified, the antagonist was added 1 minute before the inducing agent, and all the reactions were carried out at 37°C. The degree of aggregation were calculated by the following formula:

aggregation(%) = (light absorption before adding inducing agent - light absorption after adding inducing agent) / (light absorption before adding inducing agent - light absorption of Tyrode solution) x 100%

In some experiments, the compounds of formula (A) at a concentration of 100 μg/ml were found to inhibit perfectly the platelet aggregation which was induced by arachidonic acid(AA), collagen, ADP, and PAF.

Table 1 Effects of Compounds XI - 1 ~ 5 on the platelet aggregation induced by Arachidonic acid(AA), ADP, Collagen and PAF

| Compounds | concentration ($\mu$g / ml) | ADP | AA | Collagen | PAF |
|---|---|---|---|---|---|
| CONTROL | | 68.5± 3.1 | 89.8± 0.7 | 79.9± 0.7 | 87.9± 2.6 |
| XI - 1 | 100 | 1.4± 1.2$^{***}$ | 0$^{***}$ | 4.7± 3.0$^{***}$ | 0$^{***}$ |
| XI - 2 | 100 | 0$^{**}$ | 4.2± 3.4$^{***}$ | 0$^{***}$ | 0$^{***}$ |
| XI - 3 | 100 | 0$^{***}$ | 0$^{***}$ | 0$^{***}$ | 0$^{***}$ |
| XI - 4 | 100 | 2.8± 2.3$^{***}$ | 18.9±13.3$^{***}$ | 4.5± 3.9$^{***}$ | 7.7±6.3$^{***}$ |
| XI - 5 | 100 | 1.3± 1.1$^{***}$ | 0$^{***}$ | 4.7± 3.0$^{***}$ | 0$^{***}$ |

Washed rabbit platelet was incubated with each compound or 0.5 % DMSO(control) at 37 °C for 3 min., then ADP(20 $\mu$M), AA( 100 $\mu$M), Collagen(10 $\mu$g/ml) or PAF(2 ng/ml ) was added to trigger the aggregation. Percentages of aggregation are presented at mean ± S.E.M ( n = 4). * $p < 0.005$ ** $p < 0.01$ *** $p < 0.001$ as compared with the respective control value

## Claims

1. A compound of the general formula (A):

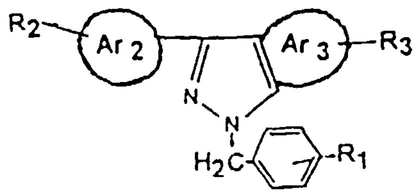

(A)

wherein

$R_1$ represents H, $C_{1-3}$alkyl, halogen, or -OR, in which R represents H or $C_{1-3}$ alkyl;

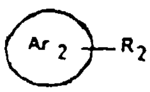 represents 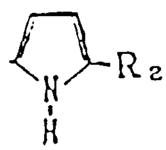 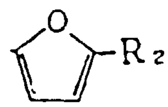 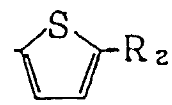 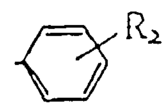

wherein
$R_2$ represents -COOR, -$CH_2$OR, H, $C_{1-3}$alkyl, or halogen, in which R is as defined above;

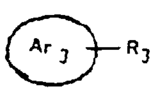 represents 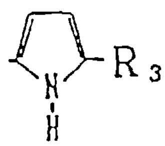 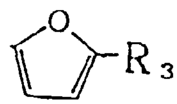 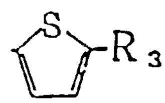 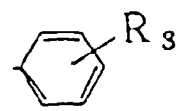

wherein
$R_3$ represents H, $C_{1-3}$alkyl, halogen, or -OR, in which R is as defined above;

and pharmaceutically acceptable salts thereof.

**2.** A compound according to Claim 1, wherein

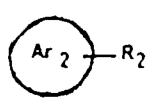 represents 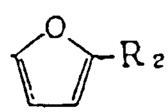

**3.** A compound according to Claim 1, wherein

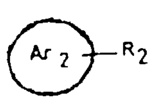 represents 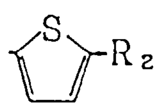

**4.** A compound according to Claim 1, wherein

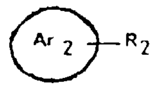 represents 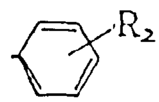

**5.** A compound according to any preceding claim, wherein

$R_1$ represents $C_{1-3}$alkyl, halogen, or -OR;
$R_2$ represents -COOR, $-CH_2OR$, $C_{1-3}$alkyl, or halogen;
$R_3$ represents H, $C_{1-3}$alkyl, halogen, or -OR;

in which R represents $C_{1-3}$alkyl.

**6.** A compound according to any one of Claims 1 to 4, wherein

$R_1$ represents $C_{1-3}$alkyl, or -OR;
$R_2$ represents -COOR, $-CH_2OR$, $C_{1-3}$alkyl, or halogen;
$R_3$ represents H, $C_{1-3}$alkyl, halogen, or -OR;

in which R represents $C_{1-3}$alkyl.

**7.** A compound according to any one of Claims 1 to 4, wherein

$R_1$ represents H, halogen, or -OR;
$R_2$ represents -COOR, $-CH_2OR$, H, or halogen;
$R_3$ represents H, halogen, or -OR;

in which R represents $C_{1-3}$alkyl.

**8.** A compound according to any one of Claims 1 to 4, wherein

$R_1$ represents H, or -OR;
$R_2$ represents -COOR, $-CH_2OR$, $C_{1-3}$alkyl, or halogen;
$R_3$ represents H, halogen, or -OR;

in which R represents $C_{1-3}$alkyl.

**9.** A process for the preparation of a compound of general formula (A), as defined in Claim 1, which comprises the steps of

(1) preparation of a substituted aryl-substituted aryl ketones compound (V).

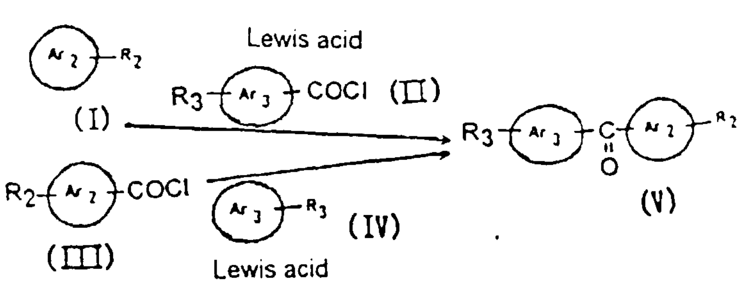


and
(2) preparation of a 1-benzyl-3-substituted aryl condensed pyrazole compound (IX)

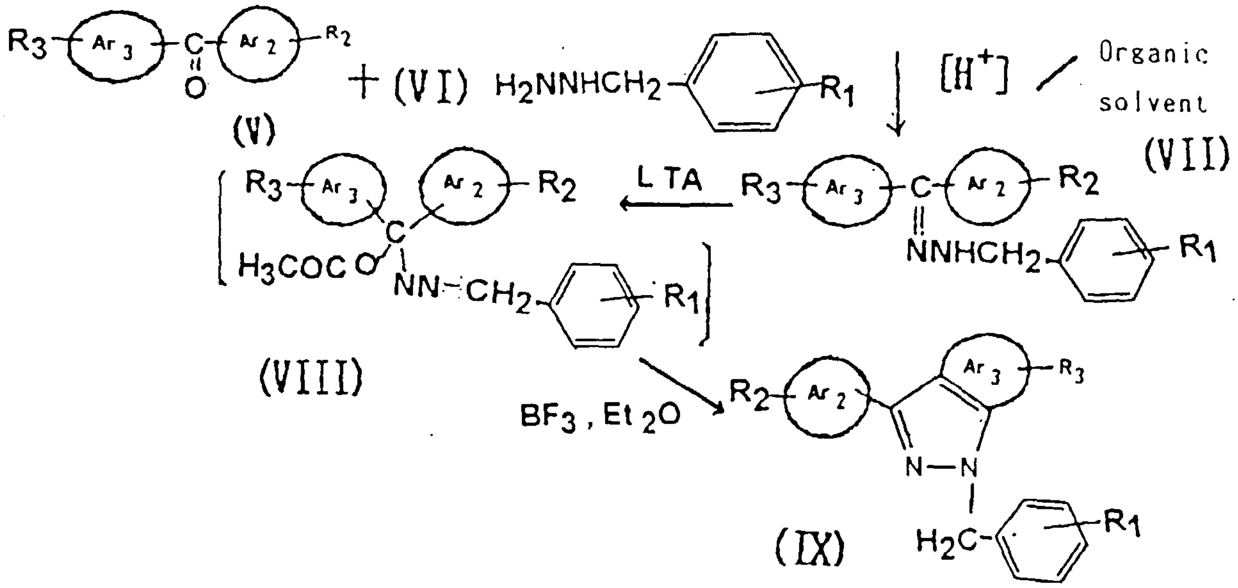


10. A process according to Claim 9 for the preparation of a compound of formula (A) in which $R_3$ represents -COOH, which comprises the further step of hydrolysis of the corresponding compound of formula (A) in which $R_3$ represents -COOR.

11. A process according to Claim 9 for the preparation of a compound of formula (A) in which $R_3$ represents $-CH_2OH$, which comprises the further step of reduction of the corresponding compound of formula (A) in which $R_3$ represents -COOR.

12. A pharmaceutical composition comprising a therepeutically effective amount a compound of general formula (A), as defined in Claim 1, in admixture with one or more pharmaceutically acceptable adjuvants.

## Patentansprüche

1. Eine Substanz der Formel (A),

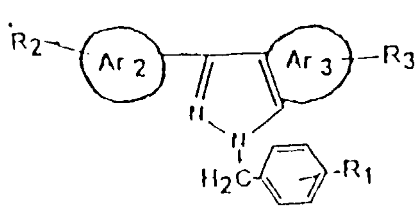

(A)

dabei steht

$R_1$ für H, $C_{1-3}$ Alkyl, Halogen oder -OR; R steht für H oder $C_{1-3}$ Alkyl;

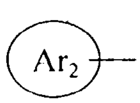 repräsentiert 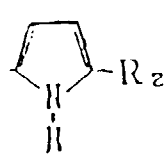 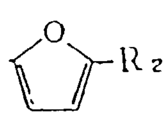 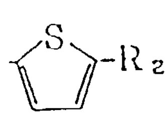 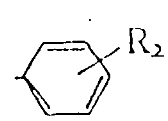

$R_2$ repräsentiert -COOR, -$CH_2$OR, H, $C_{1-3}$ Alkyl oder Halogen; R ist wie oben definiert;

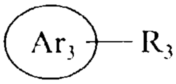 repräsentiert 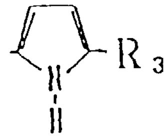 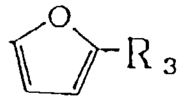 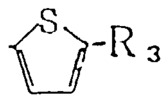 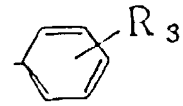

$R_3$ repräsentiert H, $C_{1-3}$ Alkyl, Halogen oder -OR; R ist wie oben definiert; Und entsprechende pharmazeutisch nutzbare Salze.

**2.** Eine Substanz entsprechend Punkt des Anspruches.

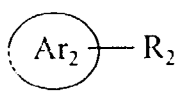 repräsentiert 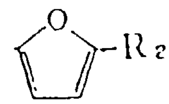

**3.** Eine Substanz entsprechend Punkt 1 des Anspruches,

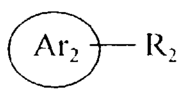 repräsentiert 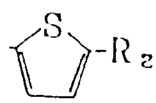

**4.** Eine Substanz entsprechend Punkt 1 des Anspruches,

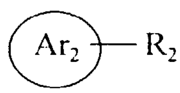 repräsentiert 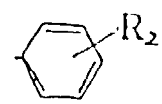

**5.** Eine Substanz entsprechend den vorangegangenen Punkten des Anspruches, wobei:

$R_1$ steht für $C_{1-3}$ Alkyl, Halogen oder -OR;
$R_2$ repräsentiert -COOR, -$CH_2$OR, $C_{1-3}$ Alkyl oder Halogen;
$R_3$ steht für H, $C_{1-3}$ Alkyl, Halogen oder -OR;
R repräsentiert $C_{1-3}$ Alkyl.

**6.** Eine Substanz entsprechend der Punkt 1 bis 4 des Anspruches, wobei:

$R_1$ repräsentiert $C_{1-3}$ Alkyl oder -OR;
$R_2$ steht für -COOR, -$CH_2$OR, $C_{1-3}$ Alkyl oder Halogen;
$R_3$ repräsentiert H, $C_{1-3}$ Alkyl, Halogen oder -OR;
R repräsentiert $C_{1-3}$ Alkyl.

**7.** Eine Substanz entsprechend der Punkte 1 bis 4 des Anspruches, wobei:

$R_1$ repräsentiert H, Halogen oder -OR;
$R_2$ steht für -COOR, -$CH_2$OR, H oder Halogen;

$R_3$ repräsentiert H, Halogen oder -OR;
R repräsentiert $C_{1-3}$ Alkyl.

**8.** Eine Substanz entsprechend der Punkte 1 bis 4 des Anspruches, wobei:

$R_1$ repräsentiert H oder -OR;
$R_2$ steht für -COOR, $-CH_2OR$, $C_{1-3}$ Alkyl oder Halogen;
$R_3$ repräsentiert H, -OR oder Halogen;
R steht für $C_{1-3}$ Alkyl.

**9.** Ein Prozess zur Herstellung von Substanzen der Formel (A)), wie in Anspruch (1) definiert, bestehend aus:

(1) Erzeugung einer substituierten Aryl -substituierten Arylketonen-Substanz V

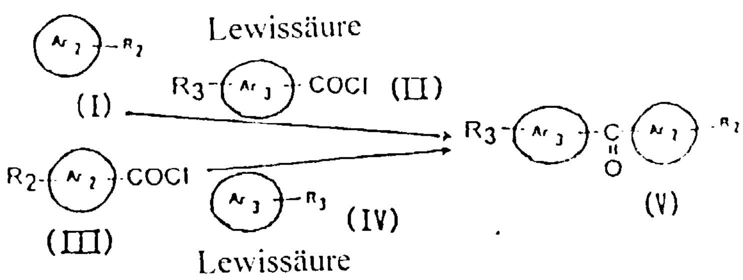


und
(2) Erzeugung eines 1-Benzyl-3-substituierten Aryl kondensierten Pyrazol (IX)

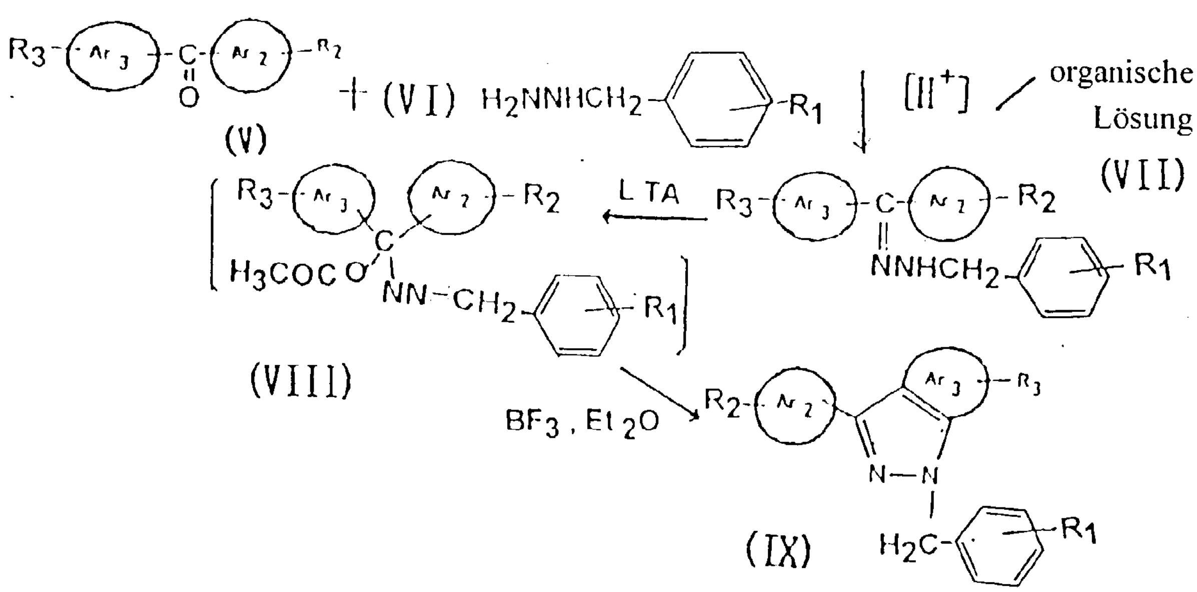


**10.** Eine entsprechend Punkt 9 des Anspruches beschriebene Methode zur Erzeugung von Stoffen der Formel (A). wobei $R_3$ für -COOH steht, desweiteren die weitere Hydrolyse der entsprechenden Substanzen der Formel (A) wobei $R_3$ für -COOR steht.

**11.** Eine entsprechend Punkt 9 des Anspruches beschriebene Methode zur Erzeugung von Stoffen der Formel (A), wobei R3 CH2OH repräsentiert, desweiteren die Reduktion der entsprechenden Substanzen der Formel (A), wobei R3 für -COOR steht.

**12.** Ein pharmazeutisch nutzbares Gemisch, welches eine für therapeutische Zwecke ausreichende Menge an Substanzen der Formel (A), wie in Anspruch (I) dargestellt, enthält, und welches mit anderen einem oder mehreren pharmazeutisch verwendbaren Beimischungen verbunden ist.

## Revendications

**1.** Un composé de formule générale (A),

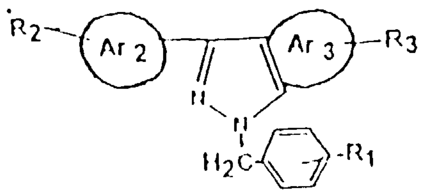

(A)

où $R_1$ représente H, $C_{1-3}$ alkyle, X (halogène), -OR, où R représente H où $C_{1-3}$ alkyle;

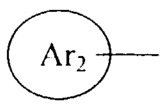 $R_2$ représente 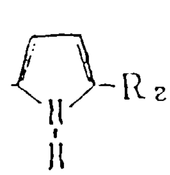 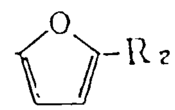 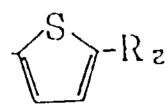 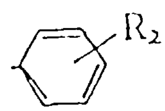

où $R_2$ représente -COOR, $-CH_2OR$, H, $C_{1-3}$ alkyle, ou halogène et où R est défini ci-dessus;

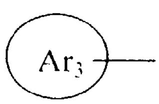 $R_3$ représente 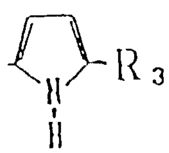 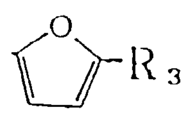 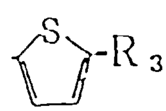 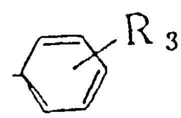

Où $R_3$ représente H, $C_{1-3}$ alkyle; où $R_3$ représente H, $C_{1-3}$ alkyle, halogène, -OR: où R est défini ci-dessus. et les sels acceptables de façon pharmaceutique ci-dessus.

**2.** Un composé de formule générale (A)selon la section 1,

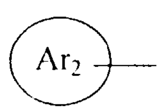 $R_2$ représente 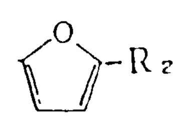

**3.** Un composé de formule générale (A), selon la section 1, où:

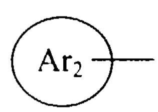 $R_2$ représente 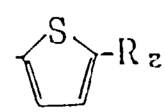

**4.** Un composé de formule générale (A), selon la section 1, où:

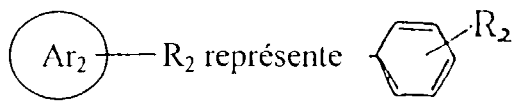


**5.** Un composé de formule générale (A), selon la section 1, où:

$R_1$ représente $C_{1-3}$ alkyle, halogène, -OR;
$R_2$ représente -COOR, $-CH_2OR$, $C_{1-3}$ alkyle, halogène;
$R_3$ représente H, $C_{1-3}$ alkyle, halogène, -OR;
OU R représente H, $C_{1-3}$ alkyle.

**6.** Un composé de formule générale (A), selon la section 1 à 4, où:

$R_1$ représente $C_{1-3}$ alkyle, -OR;
$R_2$ représente -COOR, $-CH_2OR$, $C_{1-3}$ alkyle, halogène;
$R_3$ représente H, $C_{1-3}$ alkyle, halogène, -OR;
Où R représente H, $C_{1-3}$ alkyle.

**7.** Un composé de formule générale (A), selon la section 1 à 4, où:

$R_1$ représente H, halogène, -OR;
$R_2$ représente -COOR, $-CH_2OR$, H, halogène;
$R_3$ représente H, halogène, -OR;
Où R représente H, $C_{1-3}$ alkyle.

**8.** Un composé de formule générale (A), selon la section 1 à 4, où:

$R_1$ représente H,-OR;
$R_2$ représente -COOR, -CH2OR, C1-3 alkyle, halogène;
$R_3$ représente H, -OR, halogène;
Où R représente C1-3 alkyle,H.

**9.** Un procédé de préparation d'un composé de formule générale (A)), consistant en :

(1) Une préparation des composés V de cétones d'aryle substitué

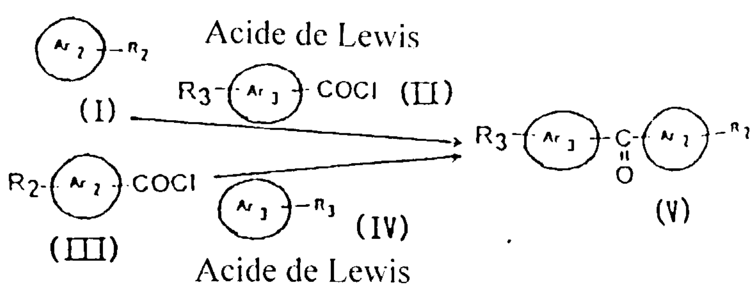


et
(2) Une préparation de pyrazoles (IX) condensés d'aryl substitué-3-benzyle-1

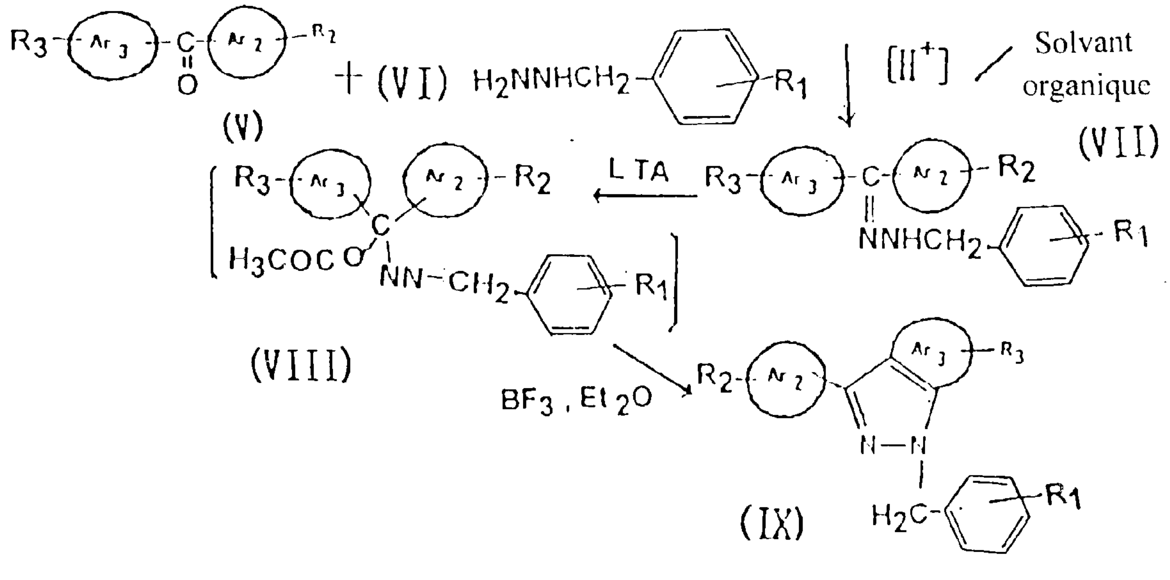


10. Le procédé selon la section 9 pour la préparation d'un composé de formule générale (A) où: $R_3$ représente -COOH, qui comprend l'étape ultérieure de l'hydrolyse du composé correspondant de formule (A) où $R_3$ représente - COOR.

11. Le procédé selon la section 9 pour la préparation d'un composé de formule générale (A) où $R_3$ représente -$CH_2OH$, qui comprend l'étape ultérieure de réduction du composé correspondant de formule (A) où $R_3$ représente -COOR.

12. La composition pharmaceutique avec une grande quantité de composés efficaces de formule générale (A), comme définis dans la Section 1, par ajout d'un ou plusieurs éléments pharmaceutiques acceptables.